# EUROPEAN PATENT APPLICATION

(11) **EP 3 733 172 A1**
(43) Date of publication of application: **04.11.2020**
(21) Application number: 20181215.3
(22) Date of filing: 24.09.2012
(51) Int. Cl.: A61K 31/194, A61K 31/198, A61K 31/555, A61K 33/06, A61K 33/26, A23L 33/16

(54) **BUFFERED UPPER GI ABSORPTION PROMOTER**

(30) Priority: 22.09.2011 US 201161538028 P
(62) Divisional of application: 12833265.7
(71) Applicant: Albion Laboratories, Inc., Layton, UT 84041 (US); Bortz, Jonathan David, St. Louis, MO 63130 (US); Hartle, Jennifer, Layton, UT 84041 (US)
(72) Inventor: BORTZ, Jonathan David, St. Louis, MO Missouri 63130 (US); HARTLE, Jennifer, Layton, UT Utah 84041 (US)
(74) Representative: Bird & Bird LLP

(57) **Abstract**

The present invention relates to compositions of an active pharmaceutical compound or nutritional ingredient with one or more buffering agents between a pH of 1.0 and 6.0, preferably 2.0 and 4.0. The buffering agents are constituted to maintain or reduce the pH of the duodenal fluid in the proximal, mid and distal duodenum. The present invention further relates to methods of improving the absorption of an iron compound or an active pharmaceutical compound within the small intestine. The present compositions and methods improve absorption or bioavailability of the administered compositions.

## Description

### Field of the Disclosure

The present invention relates to a composition of an active pharmaceutical compound or nutritional ingredient with one or more compounds with buffering capacity between a pH of 1.0 and 6.0, preferably 2.0 and 4.0. The buffering agents are constituted to maintain or reduce the pH of the duodenal fluid in the proximal, mid and distal duodenum.

### Background

The ability of iron to accept and donate electrons has made it an essential element for most forms of life because it plays a crucial role in a variety of processes, such as oxygen transport, energy production, and DNA synthesis. However, the redox activity of iron can also lead to production of oxygen free radicals, which can damage cellular components. For this reason, organisms must tightly regulate their iron levels to provide enough for their cellular needs without developing toxicity associated with iron excess.

Unlike many other nutrients, the body lacks a defined mechanism for the active excretion of iron. Therefore, body iron levels must be regulated at the point of absorption, the proximal small intestine. Much of the iron that enters the lumen of the duodenum in the diet is in the oxidized or ferric form and, therefore, must be reduced before it can be taken up by enterocytes.

### Summary

The present invention relates to compositions of an active pharmaceutical compound or a nutritional ingredient. As defined herein, a pharmaceutical composition includes compositions including a nutritional compound, a drug, or a combination thereof as the active ingredient.

As such, provided herein is a pharmaceutical composition comprising a buffering agent with efficacy between a pH of 1.0 and 6.0, and further comprising a pharmacological agent. As defined herein, the pharmacological agent can be an active pharmaceutical compound or a nutritional ingredient.

In some embodiments the pharmacological agent is nutritional ingredient. As a nutritional ingredient, the pharmacological agent can be a mineral or mineral complex. In certain embodiments the mineral or mineral complex may include a transition metal, an alkaline earth metal, an alkali metal, Fe, Ca, Mg, Mn, Zn, Se, Cu, Cr, Mo, Ni, Sn, V, B, and mixtures thereof.

In certain embodiments, the pharmacological agent is a drug selected from the group comprising an acid/alkaline-labile agent, a pH-dependent agent, or an agent that is a weak acid or a weak base, and mixtures thereof.

In another embodiment, the present disclosure relates to a pharmaceutical composition comprising a buffering agent with efficacy between a pH of 1.0 and 6.0, a pharmacological agent and an upper gastro-intestinal prokinetic agent capable of increasing upper GI transit time.

As defined herein, the pharmacological agent is an active pharmaceutical compound or a nutritional ingredient. In some embodiments the pharmacological agent is a nutritional ingredient. As a nutritional ingredient, the pharmacological agent can be a mineral or mineral complex. In certain embodiments, the pharmacological agent is a drug selected from the group comprising an acid/alkaline-labile agent, a pH-dependent agent, or an agent that is a weak acid or a weak base, and mixtures thereof.

In an additional embodiment, the present invention discloses a composition comprising a metal compound that is soluble at a pH of less than 6.0 and a buffering agent with efficacy in a pH range of 1.0-6.0.

Further disclosed is a method of improving absorption of an iron compound within the small intestine. The method comprises combining the iron compound with a buffering agent with efficacy in the pH range of 2.0-4.0 and orally administering the combination to a mammal.

In an additional embodiment, a method of treating a condition of iron deficiency is provided. The method comprises administering to a mammal in need thereof a composition comprising an iron compound which is soluble at a pH of between 1.0 and 6.0, between 2.0 and 4.0, between 1.0 and 4.0 or between 2.0 and 6.0, and a buffering agent with efficacy in the pH range of 2.0-4.0.

In some embodiments the iron compound is soluble at a pH of less than 6.0, of less than 5.0, of less than 4.0, of less than 3.0 or of less than 2.0.

In a further embodiment, the present invention relates to a method for improving the absorption of a drug selected from the group consisting of an acid-alkaline-labile drug, a pH-dependent drug, and a drug that is a weak acid or a weak base. The method comprises combining the drug with a buffering agent with efficacy in the pH range of 1.0-6.0 and administering the combination to a mammal.

In yet another embodiment, a method of treating a condition in a mammal with a drug selected from the group consisting of an acid-alkaline-labile drug, a pH-dependent drug, and a drug that is a weak acid or a weak base is provided. The method comprises administering to the mammal in need thereof a composition comprising the drug and a buffering agent with efficacy in a pH range of 1.0-6.0.

Further disclosed is a method for improving the absorption of a mineral, nutritional or pharmaceutical compound. The method comprises combining the active compound with a buffering agent with efficacy in the pH range of 1.0-6.0 and administering the combination to a mammal to achieve total absorption according to the following formula: $Total Absorption = \left\{x+\left(0.005-0.50x\right)\right\} ⋅ \left({t}_{1}+{t}_{2}\right)$where
x = baseline absorption;
t₁ = baseline compound exposure to absorptive surface area;
t₂ = additional compound exposure time to absorptive surface area by extending optimal pH in second part of duodenum; and
0.05 - 0.50 = 5% to 50% increased absorption over baseline.

In an embodiment of the present disclosure a pharmaceutical composition for oral administration to a mammal comprising a first nutritional ingredient selected from the group consisting of Sumalate®, Ferrochel@, and DimaCal®. And a first buffering agent, different from the first nutritional ingredient, with an efficacy in a pH range of 2.0 - 4.0 selected from the group consisting of Sumalate®, Ferrochel®, ferrous fumarate, ferrous sulfate, DimaCal®, succinic acid, malic acid, glycine and aspartic acid and combinations thereof.

In specific embodiments, the composition comprises between 100 and 600 milligrams of Ferrochel® if this is the first nutritional ingredient, between 100 and 600 milligrams of Sumalate® if this is the first nutritional ingredient, or between 100 and 600 milligrams of DimaCal® if this is the first nutritional ingredient.

In some instances the composition comprises a second buffering agent different from the first nutritional ingredient and the first buffering agent, the combination of the first and second buffering agents having an efficacy in a pH range of 2.0-4.0. While in other embodiments the pharmaceutical composition comprises a pharmacological agent different from the first nutritional ingredient and the first buffering agent. The pharmacological agent may be a second nutritional ingredient and the second nutritional ingredient may be a mineral or mineral complex. The second nutritional ingredients may include iron compounds consisting of iron as a salt, chelate, complex or mixtures thereof, or a calcium compounds consisting of calcium as a salt, chelate, complex or mixtures thereof.

As previously disclosed herein, a pharmacological agent may be a drug selected from the group comprising an acid/alkaline-labile agent, a pH-dependent agent, or an agent that is a weak acid or a weak base, and mixtures thereof.

Further disclosed in an embodiment is a pharmaceutical composition for oral administration to a mammal comprising between 200 - 350 milligrams Ferrochel®, between 300 - 400 milligrams malic acid and a drug selected from the group comprising an acid/alkaline-labile agent, a pH-dependent agent, or an agent that is a weak acid or a weak base, and mixtures thereof, the pharmaceutical composition increasing bioavailability of the orally administered combination from 5.0% to 50.0%.

Also disclosed is a method of improving absorption within the small intestine of a mammal comprising a combination of a first nutritional ingredient selected from the group consisting of Sumalate®, Ferrochel@, and DimaCal® with a first buffering agent selected from the group consisting of Sumalate®, Ferrochel®, ferrous fumarate, ferrous sulfate, DimaCal®, succinic acid, malic acid, glycine and aspartic acid and combination thereof, wherein the first buffering agent is different from the first nutritional agent and has efficacy in a pH range of 2.0 - 4.0, and orally administering the combination to a mammal to prolong the length of the small intestine where the duodenal fluid is maintained between a pH of 2.0 and 4.0 from 0.1 to 20 cm. In certain embodiments, composition comprises between 100 and 600 milligrams of Ferrochel@ if this is the first nutritional ingredient, between 100 and 600 milligrams of Sumalate® if this is the first nutritional ingredient, or between 100 and 600 milligrams of DimaCal® if this is the first nutritional ingredient.

In some instances the composition may comprise combining the first nutritional ingredient and the first buffering agent with a second buffering agent different from the first nutritional ingredient and the first buffering agent, the combination of the first and second buffering agents having an efficacy in a pH range of 2.0-4.0. The composition may comprise combining the first nutritional ingredient and the first buffering agent with a pharmacological agent different from the first nutritional ingredient and the first buffering agent. Depending on the embodiment, a pharmacological agent may be a drug selected from the group comprising an acid/alkaline-labile agent, a pH-dependent agent, or an agent that is a weak acid or a weak base, and mixtures thereof. The administration of a composition of the present disclosure, in certain embodiments, may increase the bioavailability of the orally administered composition from 5.0% to 50.0% within length of the small intestine where the duodenal fluid is maintained between a pH of 2.0 and 4.0.

### Brief Description of Figures

FIG. 1: Schematic representation of a portion of a human bile duct with surround organs.
FIG. 2: Schematic representation of a portion of a duodenum with surround organs.
FIG. 3: Schematic representation a duodenum with the four portions of the duodenum labeled.
FIG. 4A: X-ray of a human stomach and duodenum.
FIG. 4B: Schematic representation of a human stomach and duodenum and esophagus.
FIG. 5: Schematic representation of a cross-sectional view of a duodenum.
FIG. 6: Microscopic view of the duodenum.
FIG. 7: Schematic representation of a duodenum and stomach disclosing the normal pH zones following administration of a non-caloric, non-viscous liquid.
FIG. 8: Schematic representation of a duodenum and stomach disclosing the predicted pH zones following administration of a composition of this disclosure.
FIG. 9A: Schematic representation of the duodenum and stomach comparing FIG. 7 to FIG. 8 and disclosing the increase in the length of the duodenum that is below pH 4.0 following administration of a composition of this disclosure.
FIG. 9B: Schematic representation of the increase in the length of the duodenum that is below pH 4.0 following administration of a composition of this disclosure.

### Detailed Description

The present disclosure relates to compositions of an active pharmaceutical compound or nutritional ingredient with one or more buffering agents with efficacy between a pH of 1.0 and 6.0, preferably between 2.0 and 4.0. The buffering agents are constituted to maintain or reduce the pH of the duodenal fluid in the proximal, mid and distal duodenum. By maintaining or reducing the pH of the duodenal fluid in the desired range, bioavailability of the composition as disclosed herein is significantly improved. The active pharmaceutical or nutritional ingredients are selected from a group of agents that are soluble at acidic pH. As soluble at acidic pH, the active pharmaceutical or nutritional ingredients are optimally soluble at acidic pH but may have some solubility outside an acidic pH range. In certain embodiments, the active pharmaceutical or nutritional ingredients are soluble at a pH below 6.0. In some embodiments, the active pharmaceutical or nutritional ingredients are soluble below pH 4.0. In other embodiments, the active pharmaceutical or nutritional ingredients are soluble below pH 3.0.

In one embodiment, the nutritional ingredient is a mineral. The mineral can be any nutritional mineral, for example, a transition metal, alkaline earth metal, or alkali metal. A preferred mineral is iron or calcium.

The nutritional ingredient may include all forms. For example, iron or an iron compound may include iron with an oxidation state from -2 to 6, including ferrous and ferric forms of iron. For example, a compound can be a salt, chelate, complex, or mixtures thereof. In certain embodiments, for example, the iron compound is selected from the group consisting of ferrous sulfate, ferrous fumarate, polysaccharide iron complex, iron amino acid chelate, heme iron polypeptide, and mixtures thereof.

The active pharmaceutical compounds include drugs selected from the group consisting of an acid-alkaline-labile drug, a pH-dependent drug, a drug that is a weak acid or a weak base, and mixtures thereof. The drug can be soluble at acidic pH. As soluble at acidic pH, the drug is optimally soluble at acidic pH but may have some solubility outside an acidic pH range.

Acid-alkaline-labile drugs, pH-dependent drugs, and drugs that are a weak acid or a weak base are well known to one of ordinary skill in the art. Some examples may include antiobiotics (e.g., penicillin G, ampicillin, streptomycin, clarithromycin and azithromycin), dideoxyinosine, dideoxyadenosine, dideoxycytosine, digoxin, statins (e.g., pravastatin, fluvastatin and atorvastatin), pancreatin and bupropion. Some additional examples include, without limitation, testosterone, oxybutynin, morphine, fentanyl, lansoprazole, omeprazole, esomeprazole pantoprazole, rabeprazole, naltrexone, benzocaine, noradrenaline, isoprenaline, thiamine, atracurium, and pharmaceutically acceptable salts thereof.

Examples of additional drugs suitable for use with the present disclosure include nifedipine, emonapride, nicardipine, amosulalol, noscapine, propafenone, quinine, dipyridamole, josamycin, dilevalol, labetalol, enisoprost, metronidazole, Alendronate, Alfuzosin, Amlodipine, Amlodipine, Amphetamine, Anastrozole, Aripiprazole, Atazanavir, Atomoxetine, Atorvastatin, Azithromycin, Bevacizumab, Bicalutamide, Bisoprolol, Bosentan, Botulin toxin, Budesonide, Bupropion, Candesartan, Capecitabine, Carvedilol, Caspofungin, Cefdinir, Celecoxib, Cetirizine, Cetuximab, Ciclosporin, Ciprofloxacin, Clarithromycin, Clopidogrel, Co-amoxiclav, Darbepoetin alfa, Desloratadine, Diclofenac, Docetaxel, Donepezil, Dorzolamide, Doxazosin, Drospirenone, Duloxetine, Efavirenz, Enalapril, Enoxaparin, Erlotinib, Erythropoietin, Escitalopram, Estrogen, Eszopiclone, Etanercept, Exenatide, Ezetimibe, Factor VII, Famotidine, Fenofibrate, Fexofenadine, Filgrastim, Finasteride, Fluconazole, Fluticasone, Fluvastatin, Follitropin alfa, Follitropin beta, Gabapentin, Gemcitabine, Glatiramer, Glimepiride, Goserelin, Ibandronate, Imatinib, Imiglucerase, Infliximab, Irbesartan, Irinotecan, Lamotrigine, Latanoprost, Letrozole, Leuprolide, Levalbuterol, Levetiracetam, Levofloxacin, Levothyroxine, Lidocaine, Linezolid, Lopinavir, Loratadine, Losartan, Meloxicam, Memantine, Meropenem, Metformin, Methylphenidate, Metoprolol, Modafinil. Mometasone, Montelukast, Moxifloxacin, Mycophenolate mofetil, Niacin, Nifedipine, Olanzapine, Olmesartan, Omalizumab, Ondansetron, Orlistat, Oseltamivir, Oxaliplatin, Oxcarbazepine, Paclitaxel, Palivizumab, Paroxetine, Pemetrexed, Pioglitazone, Piperacillin, Pramipexole, Pravastatin, Pravastatin, Pregabalin, Quetiapine, Raloxifene, Ramipril, Ramipril, Ranitidine, Risedronate, Risperidone, Rituximab, Rivastigmine, Ropinirole, Rosiglitazone, Rosuvastatin, Salmeterol, Sertraline, Sevelamer, Sevoflurane, Sildenafil, Simvastatin, Somatostatin, Somatropin, Sumatriptan, Tacrolimus, Tadalafil, Tamsulosin, Tamsulosin, Tegaserod, Telmisartan, Temozolomide, Tenofovir, Terbinafine, Teriparatide, Thalidomide, Tiotropium, Tolterodine, Topiramate, Trastuzumab, Valaciclovir, Valproate semisodium, Valsartan, Vardenafil, Venlafaxine, Voglibose, Voriconazole, Ziprasidone, Zoledronate, and Zolpidem.

In certain embodiments, the compositions of the present disclosure further include an upper gastrointestinal prokinetic agent capable of increasing upper GI transit time. These prokinetic agents may be selected from a variety of agents well known in the field, for example, domperidone, benzamide, cisapride, erythromycin, itopride, metoclopramide, prucalopride, renzapride, tegaserod, mitemcinal, and mixtures thereof. Additional examples may include domperidone, benzamide, cisapride, erythromycin, itopride, metoclopramide, prucalopride, renzapride, tegaserod, mitemcinal, as well as from a group consisting of botanical prokinetic agents, herbal fruits such as Terminalia chebula, Emblica officinalis & Terminalia bellerica, herbal grasses such as Saccharum officinarum Linn., herbal rhizomes such as Zingiber officinale (ginger), Capsicurm annuum, lignans such as Elenoside, botanical blends like Hangekobokuto diacerein and mixtures thereof.

### Solubility and pH

The absorption of iron salts is very tightly coupled to the ambient pH of intestinal fluid. While inorganic iron can be absorbed through the entire length of the small intestine, the salts are only absorbed in the proximal duodenum because that is the segment of bowel in which the pH is less than 3.0. This low pH is necessary to keep the reduced form of iron in solution for absorption. Once the pH exceeds 3.0, even the more soluble ferrous form of iron precipitates and is not absorbable.

This short segment of absorbing surface area is significant, but not the only mechanism that prevents excessive intake of inorganic iron, which is primarily achieved through the pH control over solubility.

### Anatomy

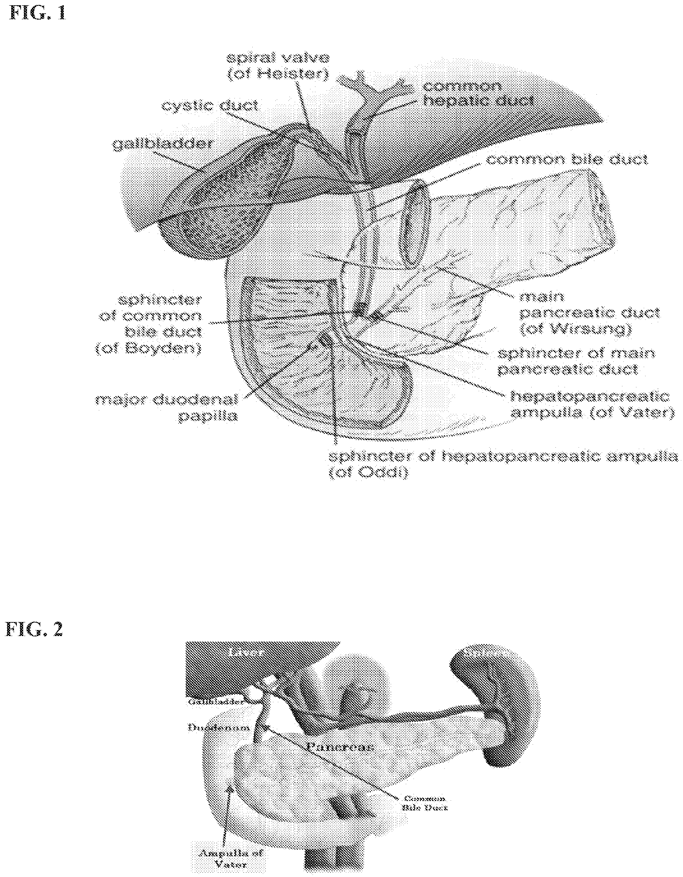

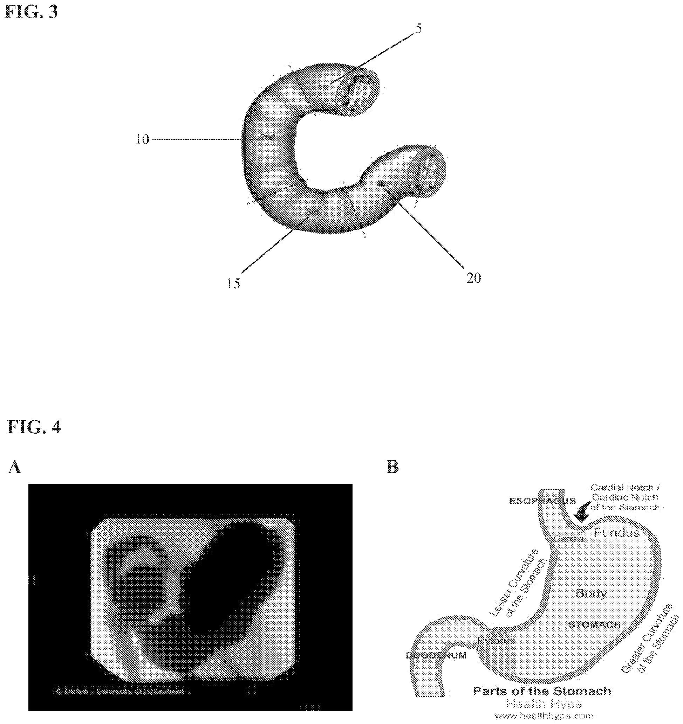

The name duodenum, meaning "two plus ten," originated because the length of this part of the small bowel was thought to be equal to 12 fingers' breadth. The general anatomy of a human duodenum and surrounding regions is represented in FIG. 1 and FIG. 2. The duodenum is the widest portion of the small bowel, and is 25-30 cm long and is divided into four sections (*see, e.g.,* FIG. 3). Referring to FIG. 3, the first (superior) portion of the duodenum **5** is about 5cm long and extends from the pylorus to the right, slightly upwards towards the neck of the gallbladder (the duodenal bulb). The second (descending) portion **10** extends for about 7.5cm from just below the neck of the gallbladder to just below the level of the 3^{rd} lumbar vertebra. The insertion of the pancreatic and biliary ducts at the ampulla of vater occurs just below the middle of the second (descending) portion **10** of the duodenum. The third (horizontal) portion **15** of the duodenum extends for about 10cm from below the third lumbar vertebra crossing in front of the aorta and inferior vena cava and below the head of the pancreas. The fourth (ascending) portion **20** extends for about 2.5cm to the ligament of Treitz at the level of the second lumbar vertebra, where it meets the body of the pancreas and turns forward as the duodenojejunal flexure.

### Physiology

Gastric acid is produced by cells lining the stomach, which are coupled to systems to increase acid production when needed. Other cells in the stomach produce bicarbonate to buffer the acid, ensuring the pH does not drop too low. Cells in the duodenum also produce large amounts of bicarbonate to completely neutralize any gastric acid that passes further down into the digestive tract. The bicarbonate-secreting cells in the stomach also produce and secrete mucus. Mucus forms a viscous physical barrier to prevent gastric acid from damaging the stomach.

The gastric pH is typically maintained at or below 1.7 under fasting conditions. The pH in the first 5.0 or 6.0 cm of the duodenum rises to between 2.0 and 3.0 and falls below pH 2.0 only sporadically and in short (5-10 second) spikes. After the ampulla of vater, the pH rises to about 5.0 with the introduction of pancreatic bicarbonate and continues to rise in the 3^{rd} and 4^{th} segment to a pH of above 6.0. Pancreatic secretions are not the only source of bicarbonate in the upper small intestine. Presence of acid in the lumen is a powerful stimulant of both gastric and duodenal HCO₃⁻ secretion. Furthermore, the secretion of bicarbonate is not the only protection that the gastric and small intestinal lumen has against the potentially ulcerative effect of gastric acid. The mucosa in the stomach and upper GI secrete mucous to create a protective layer against luminal acid.

A low pH in the duodenal lumen (∼pH 3.0 in human) causes a marked (up to fivefold) rise in the secretion of bicarbonate and the response is mediated by neural reflexes and mucosal production of prostaglandins.

### Gastro-antral-duodenal Motility

The complex and well-coordinated gastric, antral and duodenal peristaltic activity that has been observed in both solid, viscous and non-caloric, non-viscous scenarios is described below and is also key to understanding the physiologic mechanisms that underpin the present invention.

### Solid Meal

When the peristaltic wave moves over the mid-antrum-the emptying phase of the antral pump-the pyloris is opened, contractions of the duodenal bulb cease, and the proximal duodenum is relaxed. These events support the transpyloric flow. During the contractions of the terminal antrum, a peristaltic wave originates at the duodenal bulb, propelling the chyme towards the jejunum. Duodeno-gastric reflux is avoided by the simultaneous closure of the pyloris.

### Non-caloric, viscous meal

Inhibition of duodenal contractions and the start of a peristaltic wave at the duodenal bulb. This antro-pyloric-duodenal coordination is most pronounced after a non-caloric viscous meal. The duodenal peristaltic waves propagate very rapidly. The constrictions of the wave are shallow; thus the duodenal contractions do not completely empty the lumen but work like a conveyer belt.

### Non-caloric, non-viscous liquid

The antral contractions produce deep constrictions occluding the lumen when non-viscous liquids are consumed. Each peristaltic wave sweeps large quantities of liquid into the duodenum. Additionally, liquids evoke a short adaptive relaxation so that the gastric reservoir delivers the liquid to the antral pump. Consequently, non-caloric liquids empty quickly. Due to the lumen-occluding antral waves, no backflow of the liquid occurs and even during the terminal contraction, retropulsion is lacking. Thus, with non-caloric liquids, the stomach empties within a few minutes.

The relevance of the different peristaltic activities under fed (viscous and non-viscous, caloric and non-caloric) and fasting conditions is germane to the present disclosure. At the heart of this disclosure is the fact that several nutritional and pharmaceutical agents depend on the acidity of the proximal duodenal fluid for their solubility and hence bioavailability.

### Buffers

According to the present disclosure, for a buffer to be selected to maintain the optimal pH of the duodenal luminal fluid, it must have the ability to exert effective buffering activity between a pH of 2.0 and 4.0. In other words, to be effective in the pH range in which solubility is maintained for optimal absorption by the enterocytes in that relevant segment of small intestine. Those of ordinary skill in the art can select appropriate buffers for use in the present compositions. Appropriate buffers include Dicalcium malate (DimaCal®), sodium citrate, sodium phosphate, sodium acetate, or a combinations thereof. In other embodiments, ferrous bisglycinate chelate (Ferrochel®), ferrous asparto glycinate (Sumalate®), ferrous fumarate, ferrous sulfate, succinic acid, malic acid, glycine, aspartic acid can be used as the buffer in the present compositions.

In some embodiments, a single buffer comprising a single compound, e.g., Ferrochel®, is used while in other embodiments a buffer comprising combination of buffers is used. In some instances, the pharmacological agent is a nutritional supplement like Sumalate® or Ferrochel® and these ingredients may also be included as as the buffer.

In certain embodiments, Dicalcium Malate is utilized as the buffer. In other embodiments, Sumalate® or Ferrochel® or a combination thereof is utilized as the buffer. In further embodiments, a combination of Dicalcium Malate, Sumalate®, Ferrochel® or any combination thereof is used as the buffering system. In this buffering system, the Dicalcium Malate can assist in the absorption of the nutritional ingredient (e.g., iron), and the Sumalate® or Ferrochel@ can assist in the absorption of the calcium as well as the iron.

As illustrated in Table 1, Dicalcium Malate effectively exerts the desired buffering activity between a pH of 2.0 and 4.0. It has been surprisingly discovered that certain buffering agents exhibit the desired buffering characteristics. The below titration curve demonstrates why one of the most recognized buffers (calcium carbonate) in use today for human consumption, does not have the buffering characteristics that are desired according to the present disclosure. Calcium carbonate would not potentially keep the duodenal fluid acidic for an additional cm or two. According to the present disclosure, Dicalcium Malate maintains the duodenal fluid acidic for at least additional cm or two, which in reality could expand the absorptive surface in the villous duodeno-jejunal segment by more than 20%. This alone, increases the bioavailability of the accompanying mineral or pharmaceutical agent and this is the heart of the present disclosure.

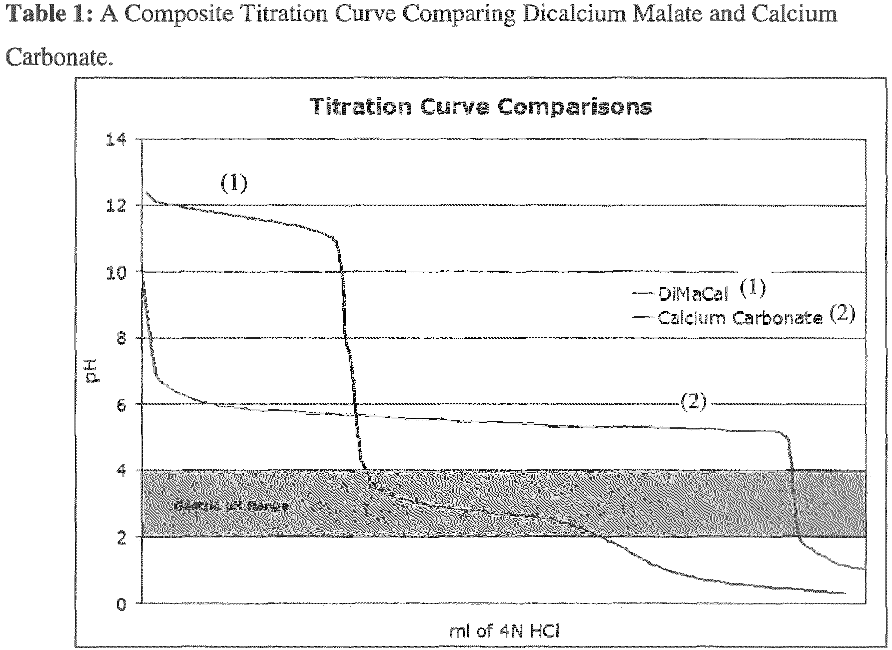

As disclosed in the composite titration curves in Table 1, Dicalcium Malate (DiMaCal) is a better buffer between a pH of 2.0 and 6.0 and specifically between a pH of 4.0 and 6.0 than calcium carbonate when measured by titration with 4N HCl.

In certain embodiments the composition comprises about 10 to 500 mg of Dicalcium Malate. In other embodiments, the composition can comprise 10 to 50 mg Dicalcium Malate, 25 to 100 mg Dicalcium Malate, 75 to 150 mg Dicalcium Malate, 125 to 200 mg Dicalcium Malate, 175 to 300 mg Dicalcium Malate, 250 to 400 mg Dicalcium Malate, or 350 to 500 mg Dicalcium Malate. Dicalcium Malate, or any other buffer or nutritional ingredient, may be present at the enumerated amounts whether utilized as a single buffer, a buffer in combination with other buffers, as a nutritional ingredient or as both a nutritional ingredient and buffer.

Unexpectedly, despite the positive titration curve discovered in Table 1, Dicalcium Malate, while effective, was not the most effective buffer for a composition of the present disclosure. Additional individual buffers or combinations of buffers have even better buffering capacity resulting is more effective adsorption of the pharmacological agent or nutritional ingredient within compositions of the present disclosure. Notably, some buffers are also pharmacological agents and nutritional ingredients.

For example, a buffer comprising Ferrochel® and malic acid, or Ferrochel® and glycine, or Ferrochel® and aspartic acid, or a combination of Ferrochel®, Dicalcium Malate, succinic acid, malic acid and glycine all have increased and unexpected buffering capacity for compositions of the present disclosure. In some embodiments, the nutritional ingredient may also be a buffer, e.g., Ferrochel® in combination with malic acid.

In a preferred embodiment, the composition comprises approximately 350 milligrams Ferrochel@ and approximately 350 milligrams malic acid. Additional preferred embodiments comprise approximately 250 milligrams Ferrochel® and approximately 250 milligrams glycine, or approximately 250 milligrams Ferrochel® and approximately 250 milligrams aspartic acid, or approximately 100 milligrams Ferrochel®, approximately 500 milligrams Dicalcium Malate and approximately 100 milligrams malic acid, or approximately 200 milligrams Ferrochel®, approximately 250 milligrams Dicalcium Malate, approximately 100 milligrams succinic acid and approximately 150 milligrams malic acid. Additional preferred embodiments contemplated by the present disclosure are disclosed in Table 12.

In certain embodiments the composition comprises about 50 to 600 milligrams Ferrochel@, about 100 to about 500 milligrams Ferrochel®, about 200 to 400 milligrams Ferrochel®. Some compositions comprise greater than 50 milligrams Ferrochel®, greater than 100 milligrams Ferrochel®, greater than 200 milligrams Ferrochel®, greater than 300 milligrams Ferrochel®, or greater than 400 milligrams Ferrochel®.

Compositions comprising malic acid may comprise between 25 and 600 milligrams malic acid, between 100 and 400 milligrams malic acid, between 200 and 300 milligrams malic acid or greater than 50 milligrams malic acid, greater than 100 milligrams malic acid, greater than 200 milligrams malic acid or greater than 300 milligrams malic acid.

Compositions comprising aspartic acid may comprise between 25 and 600 milligrams aspartic acid, between 100 and 400 milligrams aspartic acid, between 200 and 300 milligrams aspartic acid or greater than 50 milligrams aspartic acid, greater than 100 milligrams aspartic acid, greater than 200 milligrams aspartic acid or greater than 300 milligrams aspartic acid.

Compositions comprising glycine may comprise between 25 and 600 milligrams glycine between 100 and 400 milligrams glycine, between 200 and 300 milligrams glycine or greater than 50 milligrams glycine, greater than 100 milligrams glycine, greater than 200 milligrams glycine or greater than 300 milligrams glycine.

Another preferred buffer of the present disclosure is compositions comprising Sumalate® in combination with other buffering agents. For example, a composition of Sumalate® and glycine, or a composition of Sumalate® and Ferrochel@, or a composition of Sumalate®, succinic acid and malic acid. Some preferred compositions of the present disclosure comprises 250 milligrams Sumalate® and 250 milligrams glycine, or 500 milligrams Sumalate® and 250 milligrams Ferrochel®, or 250 milligrams Sumalate® and 500 milligrams Ferrochel®, or 500 milligrams Sumalate®, 142 grams succinic acid and 215 grams malic acid.

In certain embodiments the composition comprises about 50 to 600 milligrams Sumalate®, about 100 to about 500 milligrams Sumalate®, about 200 to 400 milligrams Sumalate®. Some compositions comprise greater than 50 milligrams Sumalate®, greater than 100 milligrams Sumalate®, greater than 200 milligrams Sumalate®, greater than 300 milligrams Sumalate®, or greater than 400 milligrams Sumalate®.

In some embodiments the use of ferrous fumarate or ferrous sulfate or succinic acid, independent of each other, within a composition may comprise about 50 to 600 milligrams, about 100 to about 500 milligrams, about 200 to 400 milligrams. Some compositions comprise greater than 50 milligrams, greater than 100 milligrams, greater than 200 milligrams, greater than 300 milligrams, or greater than 400 milligrams.

### Surface Area

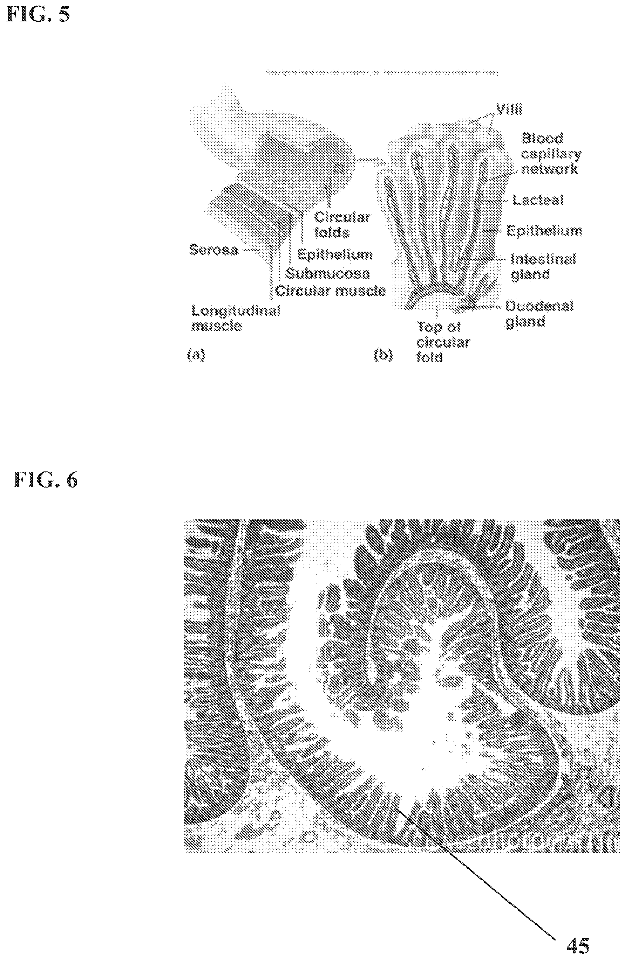

The small intestine is about 300 cm long and has an absorptive surface of up to 600m² (*see. e.g.,* FIG. 5 and FIG. 6). The duodenum is about 30 cm long and is the widest part of the small bowel and hence has an absorptive surface area of about 60m², which means that if the acidity of the duodenal fluid could be kept below 3 for just one extra cm, that would add about 2m² of absorptive area for the relevant mineral or pharmaceutical.

### pH Mapping of Duodenum

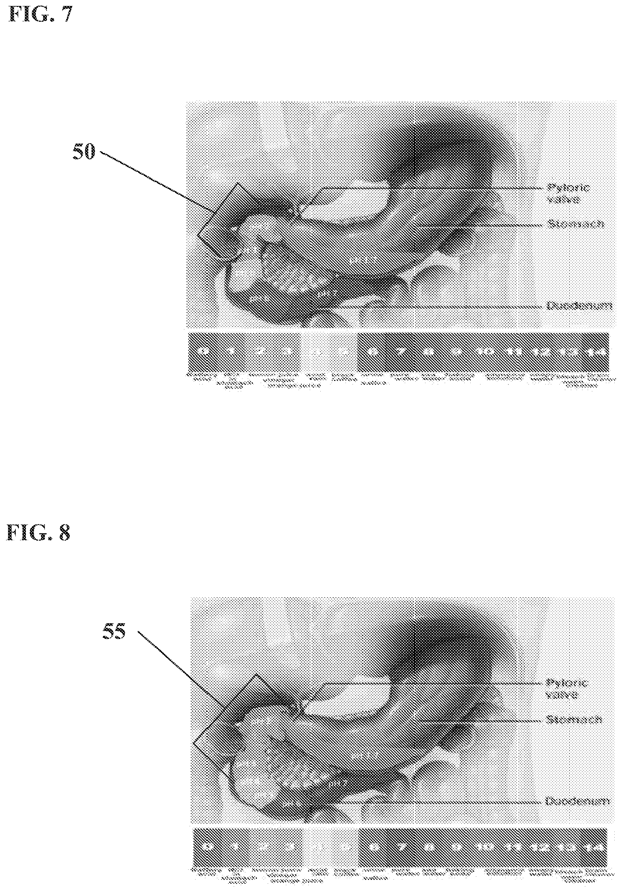

FIG. 7 is a schematic representation of a duodenum and stomach disclosing the normal pH zones following ingestion of a non-caloric, non-viscous liquid. As seen in FIG. 7, the length of duodenum that is below pH 4.0 is relatively short (*see* **50**). The region of the duodenum below pH 4.0 (**50**) is the region primarily responsible for uptake of a variety of drugs and nutritional ingredients, including iron and other metallic elements and compounds.

FIG. 8 is a schematic representation of a duodenum and stomach disclosing the predicted pH zones following administration of a composition of this disclosure, e.g., a composition comprising a buffer of Sumalate®, succinic acid and malic acid. As seen in FIG. 8, the length of duodenum that is below pH 4.0 has been extended using a presently disclosed composition (*see* **55**)*.*

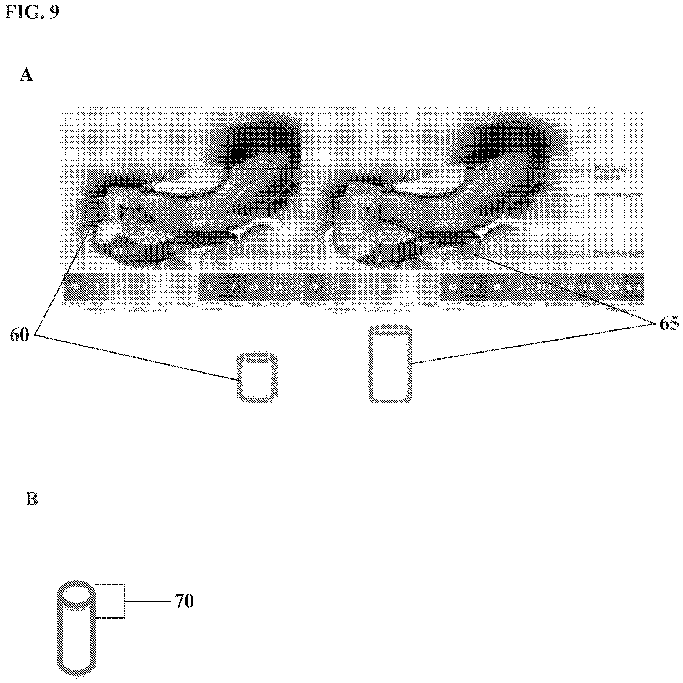

Referring now to FIG 7 and FIG. 8, while the zones of pH change in the diagrams are for illustration purposes only, the literature supports the characterization of a pH between 2.0 and 4.0 for the first 5 or 6 cm (up until approximately the ampulla of vater), and the duodenum at its distal end of having a pH of approximately 6.0. Buffers and compositions of this disclosure provide sufficient buffering capacity between a pH of 2.0 and 4.0 to be able to extend the zone of acidic duodenal fluid (compare **50** and **55**) to offer the opportunity of involving at least 10 to 20% more absorptive surface and/or stabilize the pH even in the first 5 cm (the duodenal bulb) to prevent the pH rising above 3.0 (even intermittently, as can be the case). For example, as seen in FIG. 9A, the zone for absorption under normal conditions **60** is extended upon administration of a composition of the present invention **65.**

Even a small increase in length of the duodenum that remains under pH 4.0 **70,** e.g., prior to administration of a composition of the present disclosure **60** and following administration of a composition of the present disclosure **65,** greatly increases the amount of surface for absorption. For example, every 1cm of additional length in the intestine adds 2 m² surface area **70.**

The concept of anticipating an improvement in bioavailability when exposing an iron salt to a larger surface area can be deduced from experiments performed by Hallberg and his colleagues in which he demonstrated that when the gastro-intestinal transit time is accelerated with large amounts of sorbitol or mannitol, the bioavailability of ferrous sulfate is significantly increased. This suggests that the solubilized iron salt is rapidly spread across a broader surface area before coming out of solution. (Hallberg B, Solvell, Acta. Med. Scand. (1962)17; (sup. 376)).

As disclosed herein, the presently disclosed methods increase the length of the small intestine for which the pH of the duodenal fluid is maintained between 2.0 and 4.0 by 1.0% to 10% or by 1% to 40%. In certain embodiments, the length is increased by 5.0 to 20%, increased by 7.5 to 15%, increased by 15% to 25%, increased by 20% to 30%, or increased by 25% to 40%. In other embodiments, the length is increased by greater than 1.0%, greater than 5.0%, greater than 10%, greater than 15%, greater than 20%, or greater than 25%.

In the methods as disclosed herein, the length of the duodenal segment of the small intestine for which the pH of the duodenal fluid is maintained between 2.0 and 4.0, or less than a pH of 4.0, is extended or prolonged beyond normal mammalian conditions by 0.1 to 20 cm. In some embodiments, the pH of the duodenal fluid is maintained between 1.0 and 5.0, between 2.0 and 6.0, between 2.0 and 5.0, between 3.0 and 5.0, between 3.0 and 4.0, between 3.0 and 6.0, between 1.0 and 4.0, or between 1.0 and 3.0 cm. In some embodiment, the pH of the duodenal fluid is maintained at a pH of less than 5.0, less than 4.0, less than 3.0, or less than 2.0.

In certain embodiments, the length can be between 0.1 to 10 cm, between 0.1 to 7.0 cm, between 0.1 to 5.0 cm, between 0.1 to 2.5 cm, between 0.1 to 1.5 cm, between 1.0 to 3cm, between 2.0 to 5.0 cm, between 4.0 to 7.0 cm, between 6.0 to 10 cm, between 9.0 to 14.0 cm, or between 13.0 to 20.0 cm. In some embodiments, the length can be greater than 0.1 cm, greater than 1.0 cm, greater than 1.5 cm, greater than 2.0 cm, greater than 3.0 cm, greater than 4.0 cm or greater than 5.0 cm.

Accordingly, the methods as disclosed herein can increase bioavailability by approximately 5 to 50%. In some embodiments, bioavailability is increased by greater than 3.0%, by greater than 7.0%, by greater than 10.0%, by greater than 15.0%, by greater than 20.0%, by greater than 25.0%, by greater than 35.0%, by greater than 45.0%, or by greater than 50%. In additional embodiments, the methods as disclosed herein can increase bioavailability by between 5.0% and 40.0%, by between 5.0% and 30%, by between 5.0% and 20.0%, by between 5.0% and 15.0%.

Because bioavailability of certain minerals, nutritional or pharmaceutical compounds are dependent on a specific pH range, the absorptive surface area as well as the time of exposure to the absorptive surface area, the ability of the current invention to extend the acid luminal environment, not only expands the surface area according to the quantitative model above, but it also extends the time of exposure of the compound in question to that absorptive surface area according to the following formula: $Total Absorption = \left\{x+\left(0.05-0.50x\right)\right\} ⋅ \left({t}_{1}+{t}_{2}\right)$ where:
x = baseline bioavailabililty;
t₁ = baseline compound exposure to absorptive surface area;
t₂ = additional compound exposure time to absorptive surface area by extending optimal pH in second part of duodenum; and
0.05 - 0.50 = 5% to 50% increased bioavailability based on expanding compound contact with absorptive surface area.

This range takes into account individual variations of anatomic configuration as well as the variations in acidification and alkalization of gastric and duodenal fluid, pre and postprandial conditions as well as the variability of individual compounds' pKAs.

Various modifications and alterations of this invention will become apparent to those skilled in the art without departing from the scope and spirit of the invention. Other objects and advantages will become apparent to those skilled in the art from a review of the preceding description.

### Example 1

To investigate the buffering capacity for a variety of individual buffers against neutralization in low pH levels, e.g., the duodenum, the following method was employed. 500 milligrams of each of the following individual buffers, independent of one other, were each added to 100 milliliters of distilled water: Sumalate®, Ferrochel®, ferrous fumarate, ferrous sulfate, DimaCal® (DCM), succinic acid, malic acid, glycine and aspartic acid. Each mixture was subjected to stirring to promote dissolution of the 500 milligrams. 50 milliliters of 0.1 normal HCl was added to each of the individual mixtures and then QS to 250 milliliters with distilled water. The pH of each mixture was measured. If the mixture was above pH 2.0 then the solution was lowered to a pH of 2.0 using 0.1 normal HCl. Each mixture was then titrated to a pH of 6.0 with 0.5 molar sodium bicarbonate solution. The pH for each mixture was recorded during the titration and graphed in Table 2.

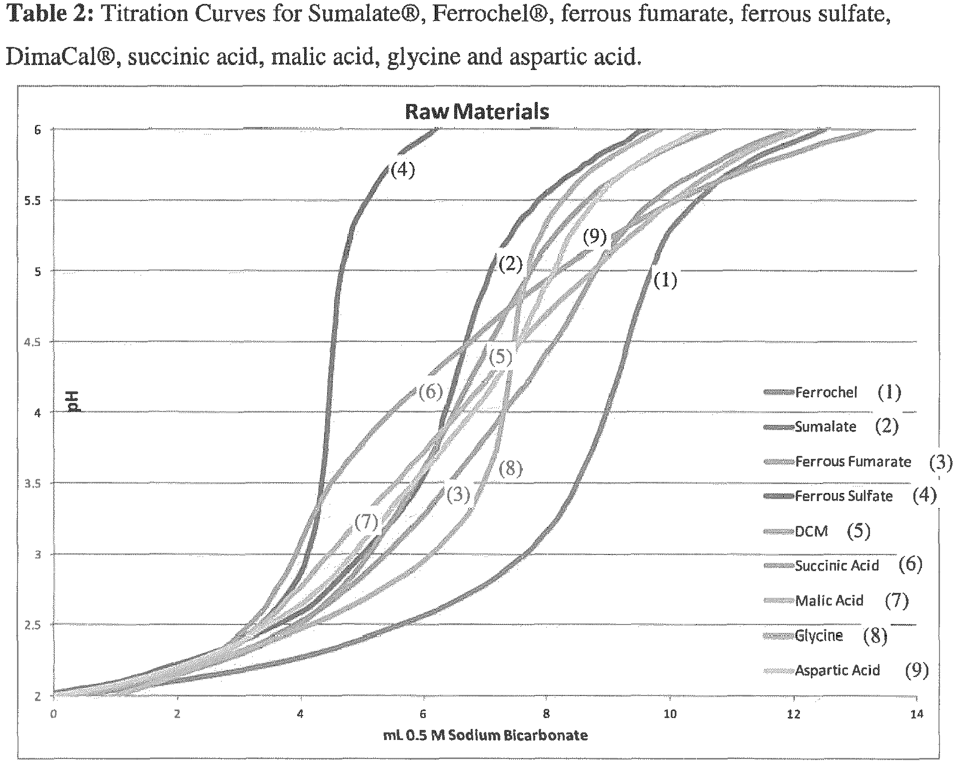

Table 2 discloses the titration curve for each individual buffer as a measure of pH on the y-axis and the amount of 0.5 M sodium bicarbonate (milliliters) on the x-axis. DimaCal® (DCM) provides buffering capacity between a pH of 2.0 and a pH of 4.0. In addition to DimaCal®, several other compounds provide buffering capacity between a pH of 2.0 and a pH of 4.0. For example, and surprisingly, Ferrochel® appears to provide the best buffering capacity compared to the other individual compounds between a pH of 2.0 and a pH of 4.0.

### Example 2

The same method as used in Example 1 was employed with a composition comprising Sumalate®, malic acid and succinic acid at different concentrations. For example, for the curve labeled "500 ratio", 500 milligrams Sumalate®, 142.86 grams of succinic acid and 214.29 grams of malic acid were added to 100 milliliters of distilled water. 50 milliliters of 0.1 normal HCl was added to the Sumalate®, malic acid, succinic acid mixture and then QS to 250 milliliters with distilled water. The pH of the Sumalate@, malic acid, succinic acid mixture was measured. If the mixture was above pH 2.0 then the mixture was lowered to a pH of 2.0 using 0.1 normal HCl for reason explained above. The Sumalate®, malic acid, succinic acid mixture was then titrated to a pH of 6.0 with 0.5 molar sodium bicarbonate solution. The pH of the Sumalate®, malic acid, succinic acid mixture was recorded during the titration and graphed in Table 3 as "500 Ratio". The same procedure was used for the "250 Ratio" and the "100 Ratio" experiments except the milligrams of each ingredient were adjusted as follows: (1) for the "250 Ratio", 250 milligrams of Sumalate®, 71.43 milligrams of succinic acid and 107.14 milligrams of malic acid were added to the original 100 milliliters of distilled water; and (2) for the "100 Ratio", 100 milligrams of Sumalate®, 28.57 milligrams of succinic acid and 42.86 milligrams of malic acid were added to the original 100 milliliters of distilled water.

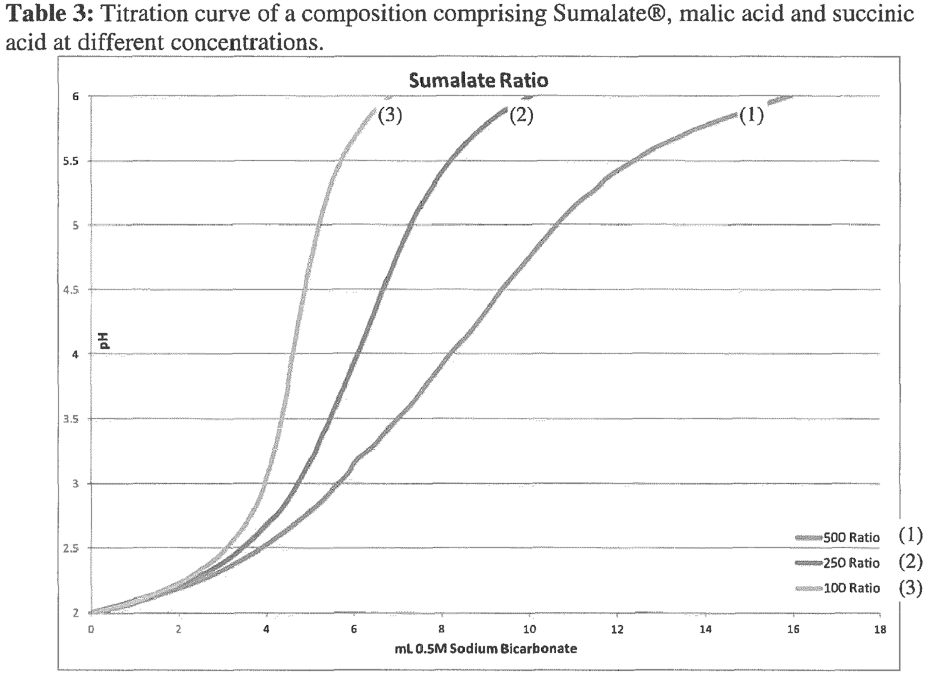

Table 3 discloses the titration curve for each ratio as a measure of pH on the y-axis and the amount of 0.5 M sodium bicarbonate (milliliters) on the x-axis. The buffering capacity between a pH of 2.0 and 4.0 increases as the concentration of the mixture increases. For example, a "500 Ratio" experiment containing 500 milligrams of Sumalate® has better buffering capacity between a pH of 2.0 and 4.0 than the composition comprising a 100 milligram of Sumalate® ("100 Ratio").

### Example 3

The same method as used in Example 1 was employed to with a composition comprising Sumalate® and various ingredients. For example, for one test buffer, 500 milligrams of Sumalate® was added to 100 milliliters of distilled water. 50 milliliters of 0.1 normal HCl was added to the Sumalate® mixture and then QS to 250 milliliters with distilled water. The pH of the Sumalate® mixture was measured. If the mixture was above pH 2.0 then the mixture was lowered to a pH of 2.0 using 0.1 normal HCl to simulate the protonation of the buffer as if it were being subjected to gastric acid. The Sumalate® mixture was then titrated to a pH of 6.0 with 0.5 molar sodium bicarbonate solution. The pH of the Sumalate® mixture was recorded during the titration and graphed in Table 4.

In another example, 350 milligrams of Sumalate®, 100 milligrams of succinic acid, and 150 milligrams of malic acid were added to 100 milliliters of distilled water. 50 milliliters of 0.1 normal HCl was added to the Sumalate®, malic acid, succinic acid mixture and then QS to 250 milliliters with distilled water. The pH of the Sumalate®, malic acid, succinic acid mixture was measured. If the mixture was above pH 2.0 then the mixture was lowered to a pH of 2.0 using 0.1 normal HCl for reason explained above. The Sumalate®, malic acid, succinic acid mixture was then titrated to a pH of 6.0 with 0.5 molar sodium bicarbonate solution. The pH of the Sumalate®, malic acid, succinic acid mixture was recorded during the titration and graphed in Table 4.

The same protocol as described above was also performed for the following additional mixtures: 350 milligrams Sumalate® and 350 milligrams malic acid; 100 milligrams Sumalate®, 500 milligrams DimaCal® and 100 milligrams malic acid; 250 milligrams Sumalate® and 250 milligrams glycine; 200 milligrams Sumalate®, 250 milligrams DimaCal®, 100 milligrams succinic acid and 150 milligrams malic acid; and 250 milligrams Sumalate® and 250 milligrams aspartic acid.

The titration curve for each mixture is graphed in Table 4.

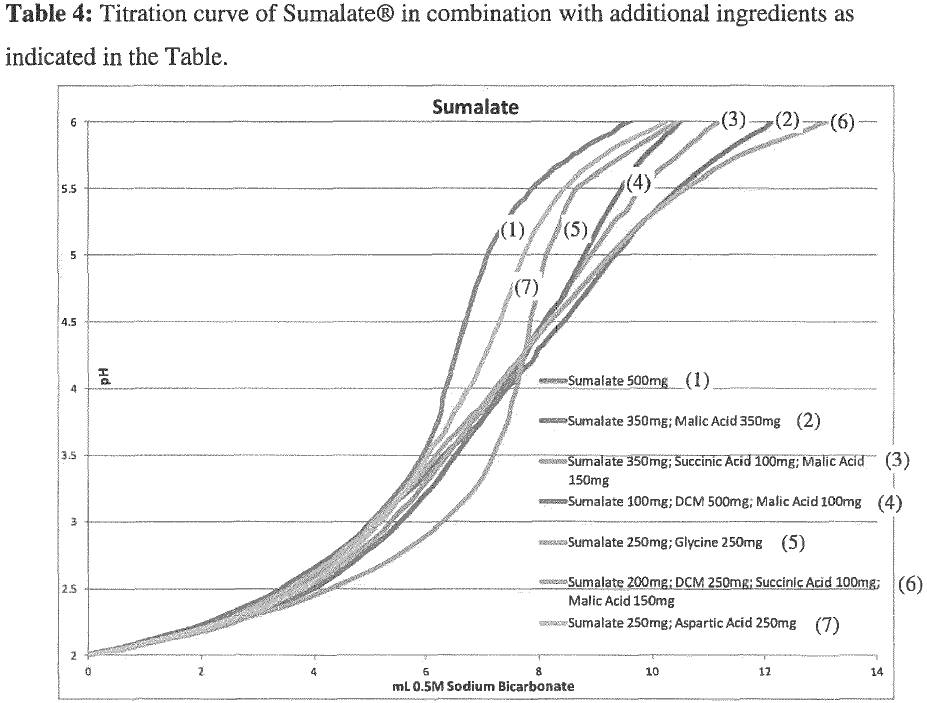

Table 4 discloses the titration curve for each composition as a measure of pH on the y-axis and the amount of 0.5 M sodium bicarbonate (milliliters) on the x-axis. The relative effectiveness of each composition to buffer is shown for each combination, for example, a composition comprising 250 milligrams of Sumalate® and 250 milligrams of glycine demonstrates the best buffering capacity between a pH of 2.0 and 4.0. And 500 milligrams of Sumalate@ without any other ingredient demonstrated the worst buffering capacity of the above-tested compositions.

### Example 4

The same method as used in Example 1 was employed with a composition comprising Ferrochel® and various ingredients. For example, for one test buffer, 500 milligrams of Ferrochel@ was added to 100 milliliters of distilled water. 50 milliliters of 0.1 normal HCl was added to the Ferrochel@ mixture and then QS to 250 milliliters with distilled water. The pH of the Ferrochel® mixture was measured. If the mixture was above pH 2.0 then the mixture was lowered to a pH of 2.0 using 0.1 normal HCl. The Ferrochel® mixture was then titrated to a pH of 6.0 with 0.5 molar sodium bicarbonate solution. The pH of the Ferrochel® mixture was recorded during the titration and graphed in Table 5.

In another example, 350 milligrams of Ferrochel®, 100 milligrams of succinic acid, and 150 milligrams of malic acid were added to 100 milliliters of distilled water. 50 milliliters of 0.1 normal HCl was added to the Ferrochel®, malic acid, succinic acid mixture and then QS to 250 milliliters with distilled water. The pH of the Ferrochel®, malic acid, succinic acid mixture was measured. If the mixture was above pH 2.0 then the mixture was lowered to a pH of 2.0 using 0.1 normal HCl. The Ferrochel®, malic acid, succinic acid mixture was then titrated to a pH of 6.0 with 0.5 molar sodium bicarbonate solution. The pH of the Ferrochel®, malic acid, succinic acid mixture was recorded during the titration and graphed in Table 5.

The same protocol as described above was also performed for the following additional mixtures: 350 milligrams Ferrochel® and 350 milligrams malic acid; 100 milligrams Ferrochel®, 500 milligrams DimaCal® and 100 milligrams malic acid; 250 milligrams Ferrochel® and 250 milligrams glycine; 200 milligrams Ferrochel@, 250 milligrams DimaCal®, 100 milligrams succinic acid and 150 milligrams malic acid; and 250 milligrams Ferrochel® and 250 milligrams aspartic acid.

The titration curve for each mixture is graphed in Table 5.

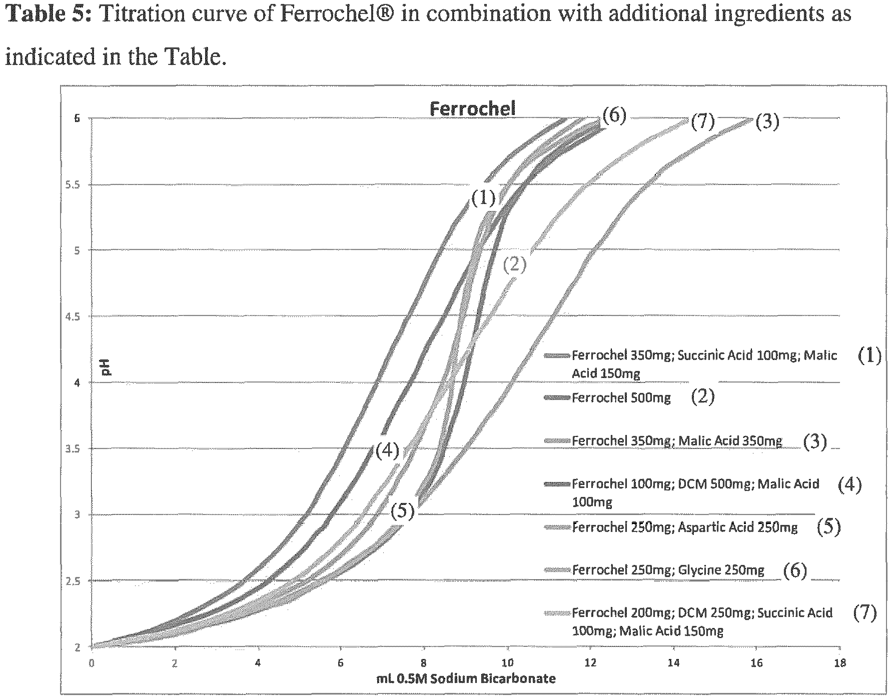

Table 5 discloses the titration curve for each composition as a measure of pH on the y-axis and the amount of 0.5 M sodium bicarbonate (milliliters) on the x-axis. The relative effectiveness of each composition to buffer is shown for each combination, for example, a composition comprising 350 milligrams of Ferrochel® and 350 milligrams of malic acid demonstrates the best buffering capacity between a pH of 2.0 and 4.0. And a composition comprising 350 milligrams of Ferrochel®, 100 milligrams of succinic acid and 150 milligrams of malic acid demonstrated the worst buffering capacity of the above-tested compositions.

### Example 5

The same method as used in Example 1 was employed with a composition comprising ferrous fumarate and various ingredients. For example, for one test buffer, 500 milligrams of ferrous fumarate was added to 100 milliliters of distilled water. 50 milliliters of 0.1 normal HCl was added to the ferrous fumarate mixture and then QS to 250 milliliters with distilled water. The pH of the ferrous fumarate mixture was measured. If the mixture was above pH 2.0 then the mixture was lowered to a pH of 2.0 using 0.1 normal HCl. The ferrous fumarate mixture was then titrated to a pH of 6.0 with 0.5 molar sodium bicarbonate solution. The pH of the ferrous fumarate mixture was recorded during the titration and graphed in Table 6.

In another example, 218.75 milligrams of ferrous fumarate, 100 milligrams of succinic acid, and 150 milligrams of malic acid were added to 100 milliliters of distilled water. 50 milliliters of 0.1 normal HCl was added to the ferrous fumarate, malic acid, succinic acid mixture and then QS to 250 milliliters with distilled water. The pH of the ferrous fumarate, malic acid, succinic acid mixture was measured. If the mixture was above pH 2.0 then the mixture was lowered to a pH of 2.0 using 0.1 normal HCl. The ferrous fumarate, malic acid, succinic acid mixture was then titrated to a pH of 6.0 with 0.5 molar sodium bicarbonate solution. The pH of the ferrous fumarate, malic acid, succinic acid mixture was recorded during the titration and graphed in Table 6.

The same protocol as described above was also performed for the following additional mixtures: 218.75 milligrams ferrous fumarate and 350 milligrams malic acid; 62.5 milligrams ferrous fumarate, 500 milligrams DimaCal® and 100 milligrams malic acid; 156.25 milligrams ferrous fumarate and 250 milligrams glycine; 125 milligrams ferrous fumarate, 250 milligrams DimaCal®, 100 milligrams succinic acid and 150 milligrams malic acid; and 156.25 milligrams ferrous fumarate and 250 milligrams aspartic acid.

The titration curve for each mixture is graphed in Table 6.

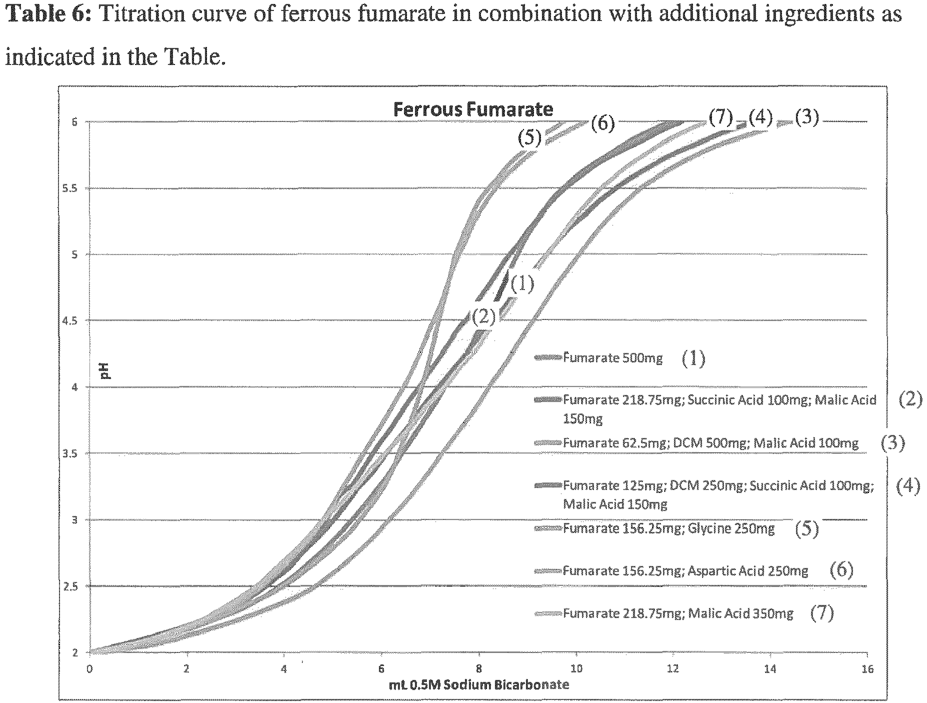

Table 6 discloses the titration curve for each composition as a measure of pH on the y-axis and the amount of 0.5 M sodium bicarbonate (milliliters) on the x-axis. The relative effectiveness of each composition to buffer is shown for each combination, for example, a composition comprising 62.5 milligrams of ferrous fumarate, 500 milligrams of DCM (DimaCal®) and 100 milligrams of malic aid demonstrates the best buffering capacity between a pH of 2.0 and 4.0. And a composition comprising 156.25 milligrams of ferrous fumarate and 250 milligrams of aspartic acid demonstrated the worst buffering capacity of the above-tested compositions.

### Example 6

The same method as used in Example 1 was employed with a composition comprising ferrous sulfate and various ingredients. For example, for one test buffer, 500 milligrams of ferrous sulfate was added to 100 milliliters of distilled water. 50 milliliters of 0.1 normal HCl was added to the ferrous sulfate mixture and then QS to 250 milliliters with distilled water. The pH of the ferrous sulfate mixture was measured. If the mixture was above pH 2.0 then the mixture was lowered to a pH of 2.0 using 0.1 normal HCl. The ferrous sulfate mixture was then titrated to a pH of 6.0 with 0.5 molar sodium bicarbonate solution. The pH of the ferrous sulfate mixture was recorded during the titration and graphed in Table 7.

In another example, 350 milligrams of ferrous sulfate, 100 milligrams of succinic acid, and 150 milligrams of malic acid were added to 100 milliliters of distilled water. 50 milliliters of 0.1 normal HCl was added to the ferrous sulfate, malic acid, succinic acid mixture and then QS to 250 milliliters with distilled water. The pH of the ferrous sulfate, malic acid, succinic acid mixture was measured. If the mixture was above pH 2.0 then the mixture was lowered to a pH of 2.0 using 0.1 normal HCl. The ferrous sulfate, malic acid, succinic acid mixture was then titrated to a pH of 6.0 with 0.5 molar sodium bicarbonate solution. The pH of the ferrous sulfate, malic acid, succinic acid mixture was recorded during the titration and graphed in Table 7.

The same protocol as described above was also performed for the following additional mixtures: 350 milligrams ferrous sulfate and 350 milligrams malic acid; 100 milligrams ferrous sulfate, 500 milligrams DimaCal® and 100 milligrams malic acid; 250 milligrams ferrous sulfate and 250 milligrams glycine; 200 milligrams ferrous sulfate, 250 milligrams DimaCal®, 100 milligrams succinic acid and 150 milligrams malic acid; and 250 milligrams ferrous sulfate and 250 milligrams aspartic acid.

The titration curve for each mixture is graphed in Table 7.

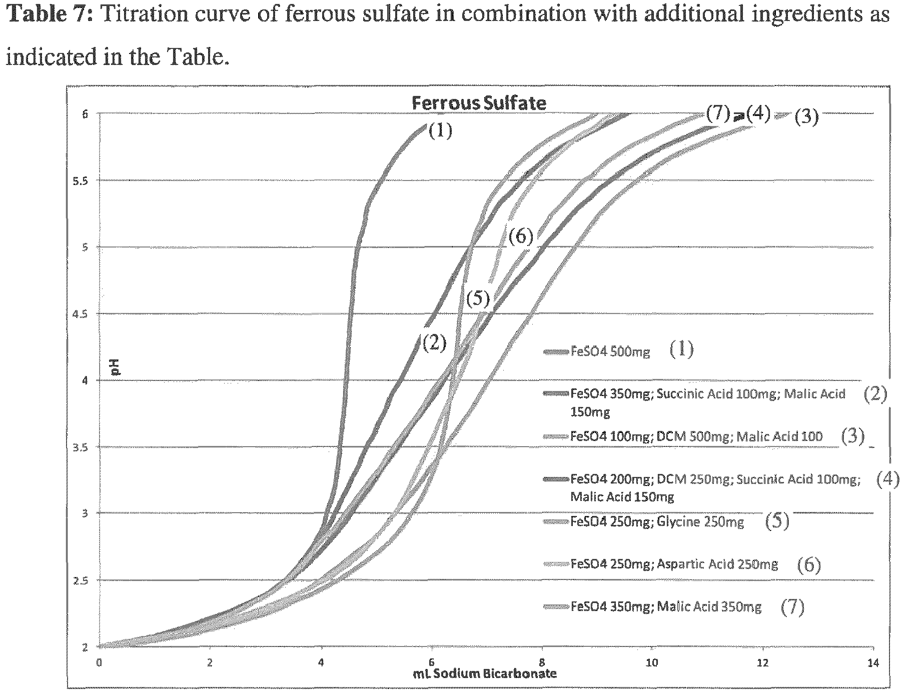

Table 7 discloses the titration curve for each composition as a measure of pH on the y-axis and the amount of 0.5 M sodium bicarbonate (milliliters) on the x-axis. The relative effectiveness of each composition to buffer is shown for each combination, for example, a composition comprising 250 milligrams of ferrous sulfate and 250 milligrams of glycine appears to demonstrate the best buffering capacity between a pH of 2.0 and 4.0. And a composition comprising 500 milligrams of ferrous sulfate demonstrated the worst buffering capacity of the above-tested compositions.

### Example 7

To understand how various sources of iron, in combination with various additional ingredients, affect the buffering capacity of a composition, the titration curves for each source of iron in combination with the same additional ingredients, as disclosed in Examples 3-6, were graphed together. The six graphs represent: 350 milligrams of iron in combination with 100 milligrams succinic acid and 150 milligrams malic acid (Table 8A); 200 milligrams iron in combination with 250 milligrams DCM (DimaCal®), 100 milligrams succinic acid and 150 milligrams malic acid (Table 8B); 100 milligrams iron in combination with 500 milligrams DCM and 100 milligrams malic acid (Table 8C); 250 milligrams iron in combination with 250 milligrams glycine (Table 8D); 250 milligrams iron in combination with 250 milligrams aspartic acid (Table 8E); and 350 milligrams iron in combination with 350 milligrams malic acid (Table 8F). The only difference between graphs within each Table (e.g., Table 8A) is the source of iron. Otherwise each composition and titration curve was prepared according to the method outlined in Example 1 and further detailed in Examples 3-6.

For example, referring to Table 8A, the titration curve for four compositions from Examples 3-6, each containing 350 milligrams of iron, 100 milligrams of succinic acid and 150 milligrams of malic acid were graphed together. The difference between each composition was the source of iron, e.g., Sumalate® (Example 3), Ferrochel@ (Example 4), ferrous fumarate (Example 5) and ferrous sulfate (Example 6).

For example, referring to Table 8B, the titration curves for four compositions from Examples 3-6, each containing 200 milligrams iron in combination with 250 milligrams DCM (DimaCal®), 100 milligrams succinic acid and 150 milligrams malic acid were graphed together. The difference between each composition was the source of iron, e.g., Sumalate® (Example 3), Ferrochel® (Example 4), ferrous fumarate (Example 5) and ferrous sulfate (Example 6). The same logic is true for Tables 8C-8F.

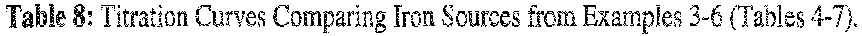

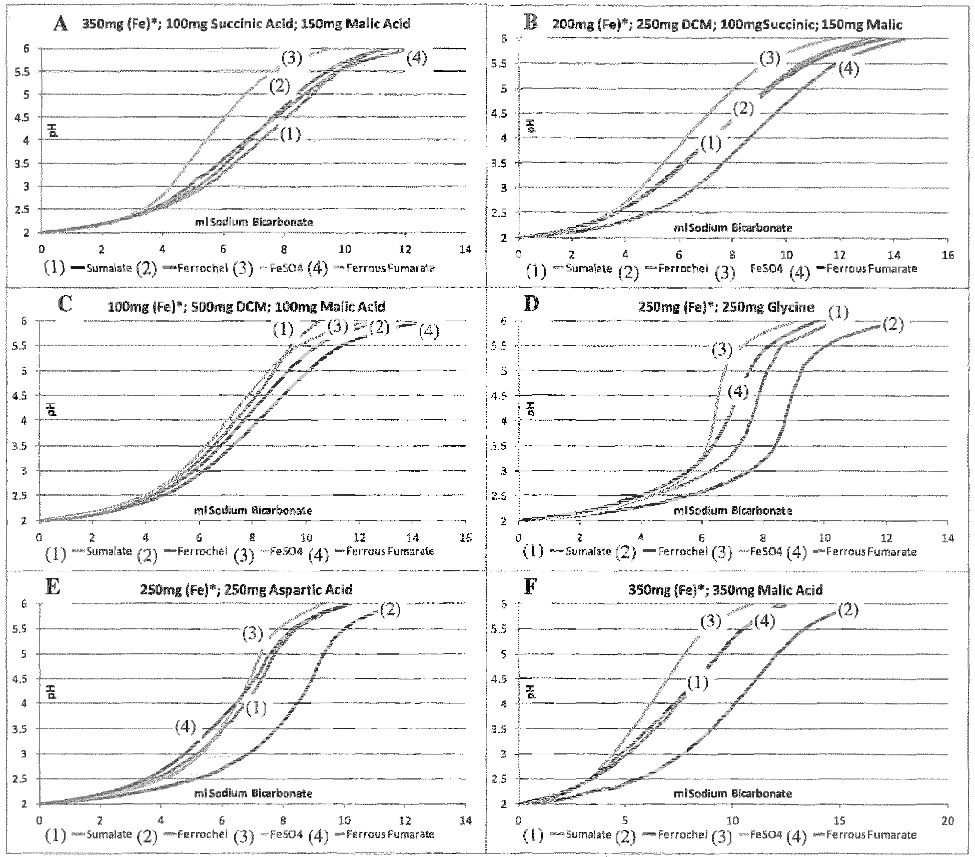

Tables 8A-8F disclose the titration curves for each composition as a measure of pH on the y-axis and the amount of 0.5 M sodium bicarbonate (milliliters) on the x-axis. Each titration curve is originally disclosed and described in Examples 3-7 but is re-disclosed here in order to compare the buffering capacity of each iron source. For example, Table 8A discloses that a composition of 350 milligrams of iron in combination with 100 milligrams succinic acid and 150 milligrams malic acid has the best buffering capacity between a pH of 2.0 and 4.0 when Sumalate® is the source of iron, and diminished buffering capacity when ferrous sulfate is the source of iron.

Table 8B discloses that a composition of 200 milligrams iron in combination with 250 milligrams DCM (DimaCal®), 100 milligrams succinic acid and 150 milligrams malic acid has the best buffering capacity between a pH of 2.0 and 4.0 when Ferrochel® is the source of iron, and diminished buffering capacity when ferrous sulfate is the source of iron. Table 8C discloses that a composition of 100 milligrams iron in combination with 500 milligrams DCM and 100 milligrams malic acid has the best buffering capacity between a pH of 2.0 and 4.0 when ferrous fumarate is the source of iron, and diminished buffering capacity when ferrous sulfate is the source of iron.

Table 8D discloses that a composition of 250 milligrams iron in combination with 250 milligrams glycine has the best buffering capacity between a pH of 2.0 and 4.0 when Ferrochel® is the source of iron, and diminished buffering capacity when ferrous sulfate is the source of iron. Table 8E discloses that a composition of 250 milligrams iron in combination with 250 milligrams aspartic acid has the best buffering capacity between a pH of 2.0 and 4.0 when Ferrochel® is the source of iron, and diminished buffering capacity when ferrous fumarate is the source of iron. Table 8F discloses that a composition of 350 milligrams iron in combination with 350 milligrams malic acid has the best buffering between a pH of 2.0 and 4.0 when Ferrochel® is the source of iron, and diminished buffering capacity when ferrous sulfate is the source of iron.

### Example 8:

To further understand the relative buffer capability between iron sources, e.g., Sumalate®, Ferrochel®, ferrous sulfate and ferrous fumarate, each iron source was combined in different ratios to examine the relative buffering between each source. Following the same method as used in Example 1, the four sources of iron were combined two at a time, at three different iron ratios each, and titrated as described in Example 1. For example, Table 9A discloses Sumalate® to Ferrochel® at an iron ratio of 1:1, 2:1 and 1:2. Table 9B discloses Sumalate® to ferrous fumarate at an iron ratio of 1:1 (250 mg:156.25 mg), 2:1 (500 mg:156.25 mg) and 1:2 (250 mg:312.5 mg). Table 9C discloses Sumalate® to ferrous sulfate at an iron ratio of 1:1 (250 mg:250 mg), 2:1 (500 mg:250 mg) and 1:2 (250 mg:500 mg). Table 9D discloses Ferrochel® to ferrous fumarate at an iron ratio of 1:1 (250 mg:156.25 mg), 2:1 (500 mg:156.25 mg) and 1:2 (250 mg:312.5 mg). Table 9E discloses Ferrochel® to ferrous sulfate at an iron ratio of 1:1 (250 mg:250 mg), 2:1 (500 mg:250 mg) and 1:2 (250 mg:500 mg). Table 9F discloses ferrous fumarate to ferrous sulfate at an iron ratio of 1:1 (156.25 mg:250 mg), 2:1 (156.25 mg:500 mg) and 1:2 (312.5 mg:250 mg).

For Tables 9A-9F, each composition was prepared according to Example 1. For example, the 1:1 composition of Sumalate® and Ferrochel@ of Table 9A was prepared by mixing equal ratios of iron from Sumalate® (250 mg) and Ferrochel® (250 mg) with 100 milliliters of distilled water. 50 milliliters of 0.1 normal HCl was added to the 1:1 Sumalate:Ferrochel® mixture and then QS to 250 milliliters with distilled water. The pH of the mixture was measured. If the mixture was above pH 2.0 then the mixture was lowered to a pH of 2.0 using 0.1 normal HCl. The mixture was then titrated to a pH of 6.0 with 0.5 molar sodium bicarbonate solution. The pH of the mixture was recorded during the titration and graphed in Table 9A. The 2:1 composition of Sumalate® to Ferrochel® mixture was prepared by mixing a 2:1 ratio of Sumalate@ (500 mg) to Ferrochel® (250 mg) with 100 milliliters of distilled water. 50 milliliters of 0.1 normal HCl was added to the 2:1 Sumalate®:Ferrochel® mixture and then QS to 250 milliliters with distilled water. The pH of the mixture was measured. If the mixture was above pH 2.0 then the mixture was lowered to a pH of 2.0 using 0.1 normal HCl. The mixture was then titrated to a pH of 6.0 with 0.5 molar sodium bicarbonate solution. The pH of the mixture was recorded during the titration and graphed in Table 9A. The 1:2 composition of Sumalate@ (250) to Ferrochel® (500 mg) mixture was prepared by mixing 1:2 ratio of Sumalate® to Ferrochel® with 100 milliliters of distilled water. 50 milliliters of 0.1 normal HCl was added to the 1:2 Sumalate®:Ferrochel® mixture and then QS to 250 milliliters with distilled water. The pH of the mixture was measured. If the mixture was above pH 2.0 then the mixture was lowered to a pH of 2.0 using 0.1 normal HCl. The mixture was then titrated to a pH of 6.0 with 0.5 molar sodium bicarbonate solution. The pH of the mixture was recorded during the titration and graphed in Table 9A.

The same procedure, with the listed iron sources, was employed to obtain the data in Tables 9B-9F.

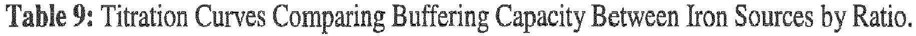

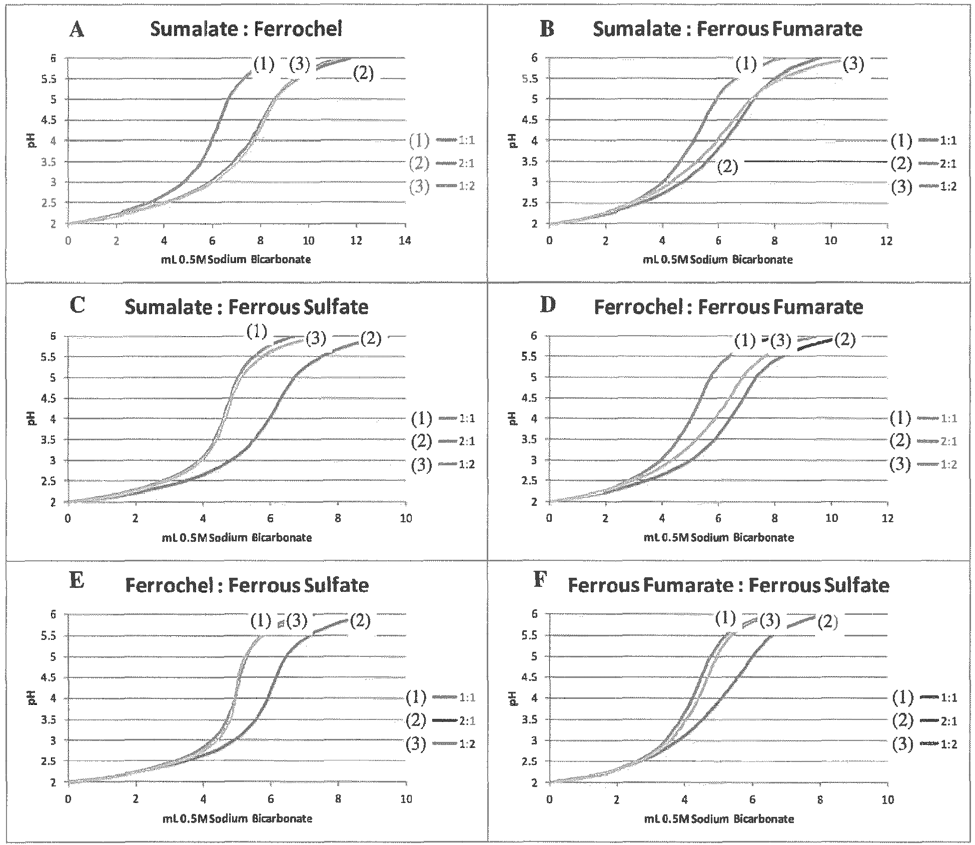

Tables 9A-9F disclose the titration curves for each composition as a measure of pH on the y-axis and the amount of 0.5 M sodium bicarbonate (milliliters) on the x-axis. As seen in Table 9A, a composition comprising a 1:2 ratio of Sumalate® to Ferrochel® or 2:1 ratio of Sumalate® to Ferrochel® has similar buffering capacity between a pH of 2.0 and 4.0, but that both ratios are better than a 1:1 ratio of Sumlate® to Ferrochel®. As seen in Table 9B, a composition comprising a 2:1 ratio of Sumalate® to ferrous fumarate has a better buffering capacity between a pH of 2.0 and 4.0 than a 1:1 ration of Sumalate® to ferrous fumarate. As seen in Table 9D, a composition comprising a ratio of 2:1 of Ferrochel® to ferrous fumarate has a better buffering capacity between a pH of 2.0 and 4.0 than a 1:1 ratio of Ferrochel® to ferrous fumarate. As seen in Table 9E, a composition comprising a ratio of 2:1 of Ferrochel® to ferrous sulfate has a better buffering capacity between a pH of 2.0 and 4.0 than a 1:1 ratio of Ferrochel® to ferrous suflate. As seen in Table 9F, a composition comprising a ratio of 2:1 of ferrous fumarate to ferrous sulfate has a better buffering capacity between a pH of 2.0 and 4.0 than a 1:1 ratio of ferrous fumarate to ferrous sulfate.

To further understand the relative buffer capability between iron sources, e.g., Sumalate®, Ferrochel®, ferrous sulfate and ferrous fumarate, the titration curves from Table 9 were regraphed in Table 10 by ratio of a single iron source against the remaining three iron sources. For example, Table 10A discloses the titration curves from Tables 9A-9C that represent a 1:1 ratio of Sumalate® with either Ferrochel®, ferrous fumarate or ferrous sulfate. Table 10B discloses the titration curves from Tables 9A-9C that represent a 2:1 ratio of Sumalate® with either Ferrochel®, ferrous fumarate or ferrous sulfate. Table 10C discloses the titration curves from Tables 9A-9C that represent a 1:2 ratio of Sumalate® with either Ferrochel®, ferrous fumarate or ferrous sulfate.

Table 10D discloses the titration curves from Tables 9A, 9D and 9E that represent a 1:1 ratio of Ferrochel® with either Sumalate®, ferrous fumarate or ferrous sulfate. Table 10E discloses the titration curves from Tables 9A, 9D and 9E that represent a2:1 ratio of Ferrochel® with either Sumalate®, ferrous fumarate or ferrous sulfate. And Table 10E discloses the titration curves from Tables 9A, 9D and 9E that represent a 1:2 ratio of Ferrochel® with either Sumalate®, ferrous fumarate or ferrous sulfate.

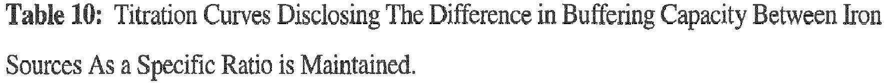

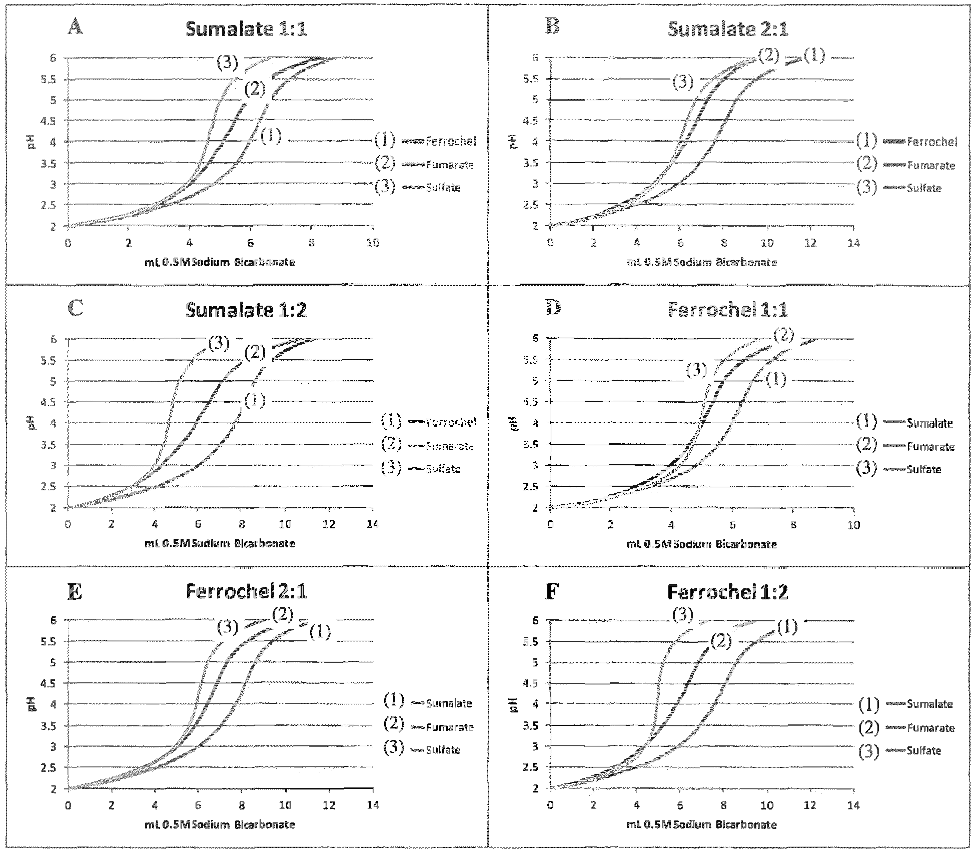

Tables 10A-10F disclose the titration curves for each composition as a measure of pH on the y-axis and the amount of 0.5 M sodium bicarbonate (milliliters) on the x-axis. As seen in Table 10A, a composition comprising a 1:1 ratio of Sumalate® to Ferrochel@ performs better as a buffer between a pH of 2.0 and 4.0 than Sumalate® in combination with either other iron source. As seen in Table 10B, a composition comprising a 2:1 ratio of Sumalate® to Ferrochel® performs better as a buffer between a pH of 2.0 and 4.0 than Sumalate® in combination with either other iron source. As seen in Table 10C, a composition comprising a 1:2 ratio of Sumalate® to Ferrochel® performs better as a buffer between a pH of 2.0 and 4.0 than Sumalate in combination with either other iron source. As seen in Tables 10D-10E, a composition comprising a 1:2, 1:1 or 2:1 ratio of Ferrochel® to Sumalate® performs better as a buffer between a pH of 2.0 and 4.0 than Ferrochel® in combination with either other iron source.

To further understand the relative buffer capability between iron sources, e.g., Sumalate®, Ferrochel®, ferrous sulfate and ferrous fumarate, the titration curves from Table 9 were regraphed in Table 11 (similar to Table 10) by ratio of a single iron source against the remaining three iron sources. For example, Table 11A discloses the titration curves from Tables 9B, 9D and 9F that represent a 1:1 ratio of ferrous fumarate with either Sumalate®, Ferrochel@ or ferrous sulfate. Table 11B discloses the titration curves from Tables 9B, 9D and 9F that represent a 1:2 ratio of ferrous fumarate with either Sumalate®, Ferrochel® or ferrous sulfate. And Table 11C discloses the titration curves from Tables 9B, 9D and 9F that represent a 2:1 ratio of ferrous fumarate with either Sumalate®, Ferrochel® or ferrous sulfate.

Table 11D discloses the titration curves from Tables 9C, 9E and 9F that represent a 1:1 ratio of ferrous sulfate with either Sumalate®, Ferrochel@ or ferrous fumarate. Table 11E discloses the titration curves from Tables 9C, 9E and 9F that represent a 1:2 ratio of ferrous sulfate with either Sumalate®, Ferrochel@ or ferrous fumarate. And Table 11F discloses the titration curves from Tables 9C, 9E and 9F that represent a 2:1 ratio of ferrous sulfate with either Sumalate®, Ferrochel® or ferrous fumarate.

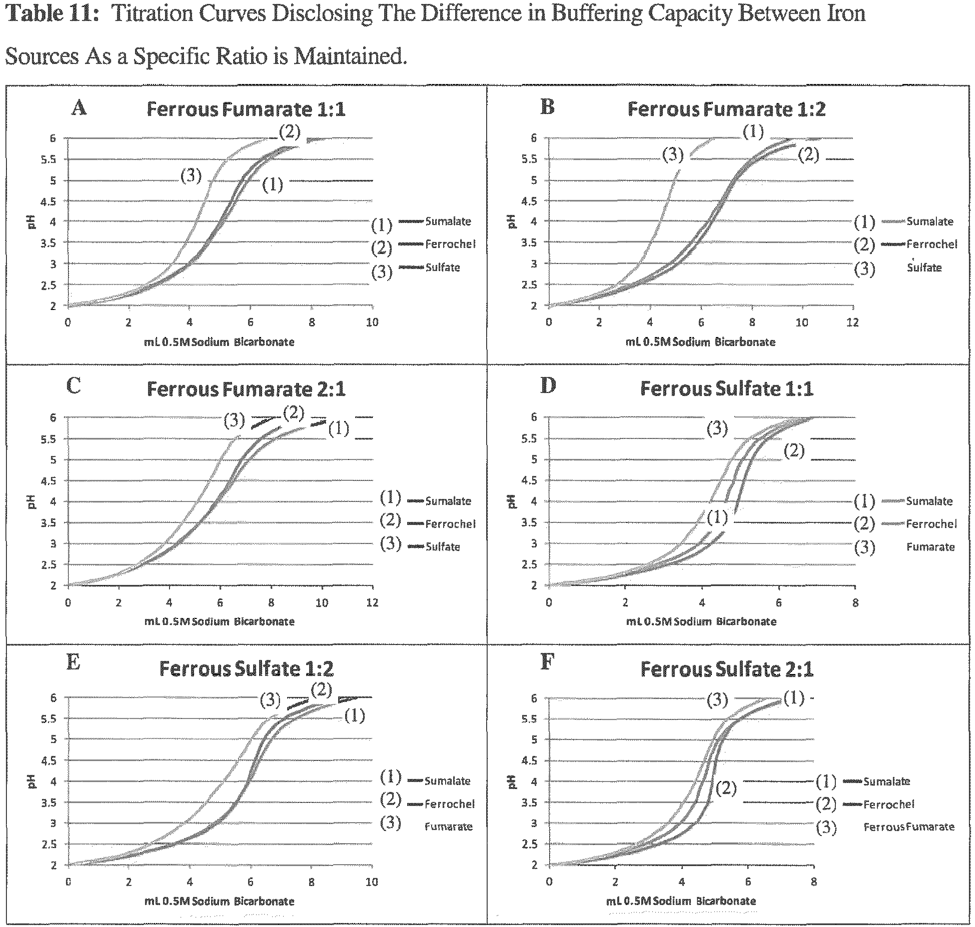

Tables 11A-11F disclose the titration curves for each composition as a measure of pH on the y-axis and the amount of 0.5 M sodium bicarbonate (milliliters) on the x-axis. As seen in Table 11A, a composition comprising a 1:1 ratio of ferrous fumarate to Ferrochel® or Sumarate® performs better as a buffer between a pH of 2.0 and 4.0 than ferrous fumarate in combination with ferrous sulfate. As seen in Table 11B, a composition comprising a1:2 ratio of ferrous fumarate to Ferrochel® or Sumarate® performs better as a buffer between a pH of 2.0 and 4.0 than ferrous fumarate in combination with ferrous sulfate. As seen in Table 11C, a composition comprising a 2:1 ratio of ferrous fumarate to Ferrochel® or Sumarate® performs better as a buffer between a pH of 2.0 and 4.0 than ferrous fumerate in combination with ferrous sulfate. As seen in Tables 11D-11E, a composition comprising a 1:1, 2:1 or 1:2 ratio of ferrous sulfate to Ferrochel® or Sumarate® performs better as a buffer between a pH of 2.0 and 4.0 than ferrous sulfate in combination with ferrous fumarate.

### Example 9:

The results of the experiments described in Examples 1 through 8 are provided below. The various compositions are listed from best buffering capacity to least buffering capacity as measured by the number of milliliters of 0.5 molar sodium bicarbonate solution required to titrate each solution to pH 6.0 from pH 2.0 as described in Example 1. The various compositions tested in Examples 3-8 are listed by number ("#") in the left hand column of Table 12. The different buffers tested are listed across the top column of Table 12, e.g., Sumalate® ("Sum"), Ferrochel® ("Fer"), ferrous fumarate ("FFum"), ferrous sulfate ("FSul"), DimaCal® ("DCM"), succinic acid ("SA"), malic acid ("MA"), glycine ("Gly") and aspartic acid ("AA"). Each buffer is listed by the number of milligrams (mg) added to any composition. The final two right hand columns entitled "Bicarb. (mls) added to ↑ pH to 3 or 4" list the number of milliliters (mls) of 0.5 molar sodium bicarbonate solution, pursuant to the method of Example 1, necessary to bring the composition from a pH of 2.0 to a pH of 3.0 or a pH of 4.0, respectively.

For example, composition number 1 comprises 350 milligrams of Ferrochel® and 350 milligrams of malic acid and it required 7.7 milliliters of 0.5 molar sodium bicarbonate solution to raise the pH of composition number 1 from a pH of 2.0 to a pH of 3.0 according to the method of Example 1. In addition, it required 10.30 milliliters of 0.5 molar sodium bicarbonate solution to the pH of composition number 1 from a pH of 2.0 to a pH of 4.0 according to the method of Example 1.

For example, composition number 5 comprises 250 milligrams of Sumarate® and 250 milligrams of glycine and it required 6.26 milliliters of 0.5 molar sodium bicarbonate solution to raise the pH of composition number 5 from a pH of 2.0 to a pH of 3.0 according to the method of Example 1. In addition, it required 7.57 milliliters of 0.5 molar sodium bicarbonate solution to the pH of composition number 1 from a pH of 2.0 to a pH of 4.0 according to the method of Example 1.

As disclosed in Table 12, the greater the number of milliliters of 0.5 molar sodium bicarbonate solution necessary to raise any given composition from a pH of 2.0 to a pH of 3.0 or 4.0, the more effective a composition comprising the listed components will be at maintaining or reducing the pH of the duodenal fluid in the proximal, mid and distal duodenum, and thereby extending the surface area available for adsorption.

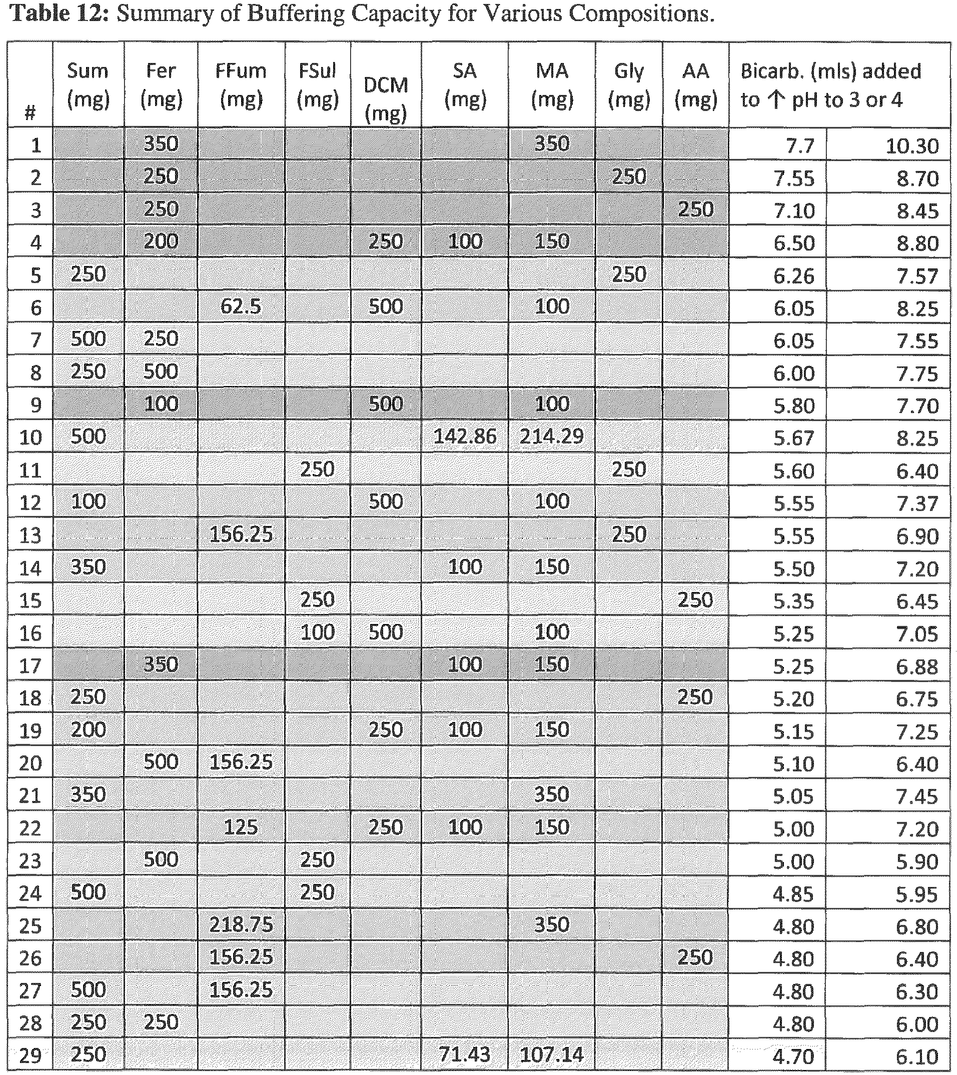

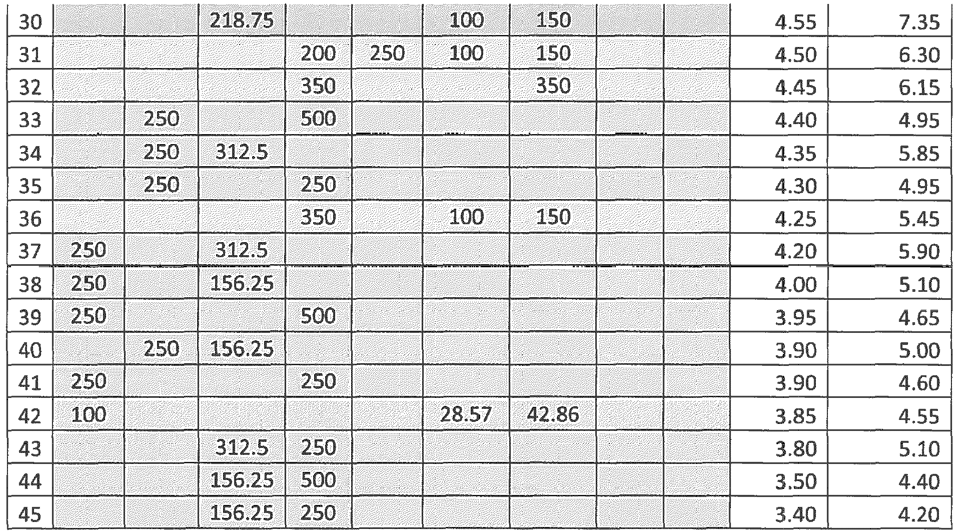

Surprisingly, the above data from Examples 3-8, as captured in Example 9 (Table 12) discloses the effectiveness of various embodiments of the present disclosure. For example, both Ferrochel® and Sumalate®, in combination with various additional ingredients, have a substantial capacity to buffer a composition between pH 2.0 and 4.0, as disclosed by the number of milliliters of 0.5 molar sodium bicarbonate solution necessary to raise the pH of composition numbers 1-10 from a pH of 2.0 to a pH of 3.0 or 4.0. In addition, this data also discloses the effectiveness of various other embodiments of the present disclosure, e.g., DCM in combination with other ingredients. These results indicate that compositions contemplated by the present disclosure, e.g., as disclosed in Table 12, have the ability to extend the length of the duodenum where the pH is below 4.0 thereby increasing the potential update of a pharmacological or nutritional agent, e.g., iron.

### Aspects

1. A pharmaceutical composition for oral administration to a mammal comprising:
   a first nutritional ingredient selected from the group consisting of Sumalate(R), Ferrochel(R), and DimaCal(R); and
   a first buffering agent, different from the first nutritional ingredient, with an efficacy in a pH range of 2.0 - 4.0 selected from the group consisting of Sumalate(R), Ferrochel(R), ferrous fumarate, ferrous sulfate, DimaCal(R), succinic acid, malic acid, glycine and aspartic acid and combinations thereof.
2. The pharmaceutical composition of aspect 1, wherein the composition comprises between 100 and 600 milligrams of Ferrochel(R) if this is the first nutritional ingredient, between 100 and 600 milligrams of Sumalate(R) if this is the first nutritional ingredient, or between 100 and 600 milligrams of DimaCal(R) if this is the first nutritional ingredient.
3. The pharmaceutical composition of aspect 1, further comprising a second buffering agent different from the first nutritional ingredient and the first buffering agent, the combination of the first and second buffering agents having an efficacy in a pH range of 2.0-4.0.
4. The pharmaceutical composition of aspect 1, further comprising a pharmacological agent different from the first nutritional ingredient and the first buffering agent.
5. The pharmaceutical composition of aspect 4, wherein the pharmacological agent is a second nutritional ingredient.
6. The pharmaceutical composition of aspect 5, wherein the second nutritional ingredient is a mineral or mineral complex.
7. The pharmaceutical composition of aspect 6, wherein the second nutritional ingredient is an iron compound consisting of iron as a salt, chelate, complex or mixtures thereof.
8. The pharmaceutical composition of aspect 6, wherein the second nutritional ingredient is a calcium compound consisting of calcium as a salt, chelate, complex or mixtures thereof.
9. The pharmaceutical composition of aspect 4, wherein the pharmacological agent is a drug selected from the group comprising an acid/alkaline-labile agent, a pH-dependent agent, or an agent that is a weak acid or a weak base, and mixtures thereof.
10. The pharmaceutical composition for oral administration to a mammal comprising:
   between 200 - 350 milligrams Ferrochel(R);
   between 300 - 400 milligrams malic acid; and
   a drug selected from the group comprising an acid/alkaline-labile agent, a pH-dependent agent, or an agent that is a weak acid or a weak base, and mixtures thereof,
   the pharmaceutical composition increasing bioavailability of the orally administered combination from 5.0% to 50.0%.
11. A method of improving absorption within the small intestine of a mammal comprising:
   combining a first nutritional ingredient selected from the group consisting of Sumalate(R), Ferrochel(R), and DimaCal(R) with a first buffering agent selected from the group consisting of Sumalate(R), Ferrochel(R), ferrous fumarate, ferrous sulfate, DimaCal(R), succinic acid, malic acid, glycine and aspartic acid and combinations thereof, wherein the first buffering agent is different from the first nutritional agent and has an efficacy in a pH range of 2.0 - 4.0; and
   orally administering the combination to a mammal to prolong the length of the small intestine where the duodenal fluid is maintained between a pH of 2.0 and 4.0 from 0.1 to 20 cm.
12. The method of aspect 11, wherein the combination comprises between 100 and 600 milligrams of Ferrochel(R) if this is the first nutritional ingredient, between 100 and 600 milligrams of Sumalate(R) if this is the first nutritional ingredient, or between 100 and 600 milligrams of DimaCal(R) if this is the first nutritional ingredient.
13. The method of aspect 11, further comprising combining the first nutritional ingredient and the first buffering agent with a second buffering agent different from the first nutritional ingredient and the first buffering agent, the combination of the first and second buffering agents having an efficacy in a pH range of 2.0-4.0.
14. The method of aspect 11, further comprising combining the first nutritional ingredient and the first buffering agent with a pharmacological agent different from the first nutritional ingredient and the first buffering agent.
15. The method of aspect 14, wherein pharmacological agent is a drug selected from the group comprising an acid/alkaline-labile agent, a pH-dependent agent, or an agent that is a weak acid or a weak base, and mixtures thereof.
16. The method of aspect 15, wherein the length of the small intestine where the duodenal fluid is maintained between a pH of 2.0 and 4.0, increases bioavailability of the orally administered combination from 5.0% to 50.0%.

## Claims

1. A pharmaceutical composition for oral administration to a mammal comprising:
a first nutritional ingredient consisting of 50 to 600 milligrams of dicalcium malate;
a second nutritional ingredient consisting of a mineral or mineral complex that includes magnesium; and
a first buffering agent selected from the group consisting of ferrous bisglycinate chelate, ferrous asparto glycinate, ferrous fumarate, ferrous sulfate, succinic acid, malic acid, glycine, aspartic acid, and combinations thereof.

2. The pharmaceutical composition according to claim 1, wherein said pharmaceutical composition further comprises a pharmacological agent different from the first nutritional ingredient, the second nutritional ingredient, and the first buffering agent, wherein the pharmacological agent is
(i) a third nutritional ingredient that is a mineral complex or
(ii) a drug selected from the group comprising an acid/alkaline-labile agent, a pH-dependent agent, or an agent that is a weak acid or a weak base, and mixtures thereof.

3. The pharmaceutical composition of claim 1, further comprising a second buffering agent different from the first nutritional ingredient, the second nutritional ingredient, and the first buffering agent, the combination of the first and second buffering agents having an efficacy in a pH range of 2.0-4.0.

4. The pharmaceutical composition of claim 2, wherein the third nutritional ingredient is an iron compound consisting of iron as a salt, chelate, complex or mixtures thereof.

5. The pharmaceutical composition of claim 2, wherein the third nutritional ingredient is a calcium compound consisting of calcium as a salt, chelate, complex or mixtures thereof.

6. The pharmaceutical composition according to claim 1 comprising:
between 50 to 600 milligrams of dicalcium malate;
a second nutritional ingredient consisting of a mineral or mineral complex that includes magnesium;
between 25 - 600 milligrams glycine; and
a drug selected from the group comprising an acid/alkaline-labile agent, a pH-dependent agent, or an agent that is a weak acid or a weak base, and mixtures thereof,
the pharmaceutical composition increasing bioavailability of the orally administered combination from 5.0% to 50.0%.

7. Pharmaceutical compositions according to any one of the preceding claims, wherein said pharmaceutical composition is orally administered.
